# EUROPÄISCHE PATENTSCHRIFT

(11) **EP 0 740 669 B1**
(45) Veröffentlichungstag und Bekanntmachung des Hinweises auf die Patenterteilung: **28.04.1999**
(21) Anmeldenummer: 95906294.4
(22) Anmeldetag: 04.01.1995
(51) Int. Cl.: C07D 498/10, C07D 265/04, A01N 43/76

(54) **AZATRIOXASPIROALKENE ALS SCHÄDLINGSBEKÄMPFUNGSMITTEL**
AZATRIOXASPIROALKENES AS PARASITICIDES
AZATRIOXASPIROALCENES UTILISES COMME PARASITICIDES

(30) Priorität: 17.01.1994 DE 4401105; 02.09.1994 DE 4431225
(43) Veröffentlichungstag der Anmeldung: 06.11.1996
(73) Patentinhaber: BAYER AG, 51368 Leverkusen (DE)
(72) Erfinder: FISCHER, Reiner, D-40789 Monheim (DE); WACHENDORFF-NEUMANN, Ulrike, D-40789 Monheim (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: EP9500023
(87) Internationale Veröffentlichungsnummer: WO9519364

(56) Entgegenhaltungen:
- EP-A- 0 345 775
- EP-A- 0 432 661
- EP-A- 0 553 623
- WO-A-93/21165
- WO-A-93/24470
- CHEMICAL ABSTRACTS, vol. 80, no. 15, 15.April 1974 Columbus, Ohio, US; abstract no. 82842q, N.A. SOKOLOV, L.S. NEMTSOVA: "Properties of nitrodioxanes" Seite 392; XP002050733 & VESTSI AKAD. NAVUK BELARUS. SSR. SER. KHIM. NAVUK, Bd. 6, 1973, Seiten 78-81,
- CHEMICAL ABSTRACTS, vol. 104, no. 1, 6.Januar 1986 Columbus, Ohio, US; abstract no. 5233d, B.O. KRAIZ: "Conformation of 5,5-disubstituted 2,2-dimethyl-1,3-dioxanes" Seite 476; XP002050734 & KHIM. GETEROTSIKL. SOEDIN., Bd. 4, 1986, Seiten 468-473,
- CHEMICAL ABSTRACTS, vol. 112, no. 17, 23.April 1990 Columbus, Ohio, US; abstract no. 157392h, E. KLEINPETER ET AL.: "Conformational arrangement of dipolar substituents in the 5-position of 1,3-dioxanes" Seite 641; XP002050735 & MONATSH. CHEM., Bd. 120, Nr. 8/9, 1989, Seiten 725-733,

## Beschreibung

Die Erfindung betrifft neue Azatrioxaspiroalkene, mehrere Verfahren zu ihrer Herstellung und ihre Verwendung-zur Bekämpfung von tierischen Schädlingen.

Es ist bereits bekannt, daß bestimmte Oxazolinderivate insektizide und akarizide Eigenschaften aufweisen (vgl. z.B. EP-A 0345775 und EP-A 0432661).

Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen Azatrioxaspiroalkene der Formel (I) gefunden, 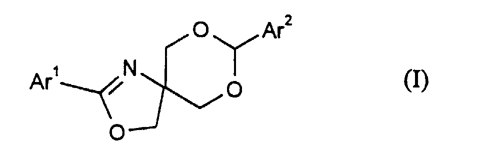 in welcher
- Ar¹ und Ar²: gleich oder verschieden sind und unabhängig voneinander jeweils für gegebenenfalls substituiertes Aryl stehen.

Weiter wurde gefunden, daß man Azatrioxaspiroalkene der Formel (I) erhält, wenn man
(a) Aminoalkohole der Formel (II) 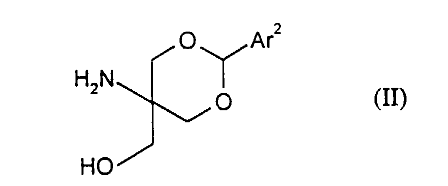 in welcher
   - Ar²: die oben angegebene Bedeutung hat,
   mit Carbonsäuren der Formel (III)

   Ar¹-COOH (III)

   in welcher
   - Ar¹: die oben angegebene Bedeutung hat,
   mit einem wasser-entziehenden Mittel gegebenenfalls in Gegenwart eines Verdünnungsmittels umsetzt, oder wenn man
(b) Amidalkohole der Formel (IV) 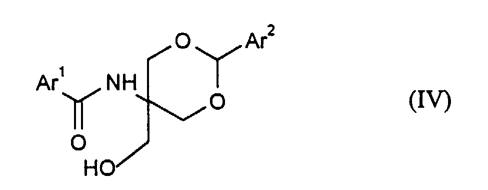 in welcher
   - Ar¹ und Ar²: die oben angegebene Bedeutung haben,
   mit einem wasser-entziehenden Mittel gegebenenfalls in Gegenwart eines Verdünnungsmittels umsetzt, oder wenn man
(c) Carbonsäureamide der Formel (V) 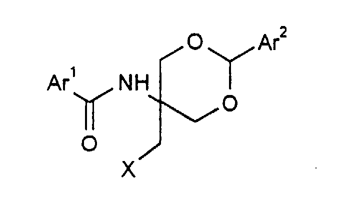 in welcher
   - Ar¹ und Ar²: die oben angegebene Bedeutung haben und
   - X: für Halogen, Alkylsulfonyloxy oder Arylsulfonyloxy steht,
   mit einer Base gegebenenfalls in Gegenwart eines Verdünnungsmittels umsetzt oder wenn man
d) Carboxamidbenzoate der Formel (VI) 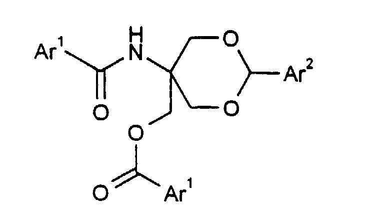 in welcher
   - Ar¹ und Ar²: die oben angegebene Bedeutung haben,
   mit einer Base gegebenenfalls in Gegenwart eines Verdünnungsmittels umsetzt.

Weiterhin wurde gefunden, daß Azatrioxaspiroalkene der Formel (I) sehr gut zur Bekämpfung von tierischen Schädlingen geeignet sind. Sie zeichnen sich insbesondere durch hohe Wirksamkeit gegen Arthropoden und Nematoden aus. Die Azatrioxaspiroalkene der Formel (I) können als Stereoisomerengemisch wie auch in Form ihrer reinen E- oder Z-Isomeren eingesetzt werden.

Überraschenderweise zeigen die erfindungsgemäßen Azatrioxaspiroalkene der Formel (I) eine erheblich bessere Wirksamkeit gegenüber tierischen Schädlingen als die konstitutionell ähnlichsten vorbekannten Verbindungen.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- Ar¹: steht vorzugsweise für Phenyl, das gegebenenfalls einfach bis fünffach, gleich oder verschieden substituiert ist durch Halogen, Cyano, Nitro, Hydroxy, C₁-C₆-Alkyl_{,} C₁-C₆-Alkoxy, C₁-C₆-Alkylthio, C₁-C₆-Hälogenälkyl, C₁-C₆-Halogenalkoxy oder C₁-C₆-Halogenalkylthio,
- Ar²: steht vorzugsweise für Phenyl, das gegebenenfalls einfach bis fünffach, gleich oder verschieden substituiert ist durch Halogen, Cyano, Nitro, Hydroxy, C₁-C₁₈-Alkyl, C₁-C₈-Halogenalkyl, C₁-C₈-Alkoxy-C₁-C₈-alkyl, C₁-C₁₈-Alkoxy (bei dem gegebenenfalls einzelne Methylengruppen durch Sauerstoffatome ersetzt sind), C₁-C₈-Halogenalkoxy, C₁-C₁₈-Alkylthio, C₁-C₈-Halogenalkylthio, C₁-C₈-Alkyl-carbonyl, C₁-C₈-Halogenalkyl-carbonyl, C₁-C₈-Alkoxycarbonyl, C₁-C₈-Halogenalkoxy-carbonyl, C₁-C₃-Alkylendioxy, C₁-C₃-Halogenalkylendioxy, jeweils gegebenenfalls durch C₁-C₆-Alkyl, C₁-C₆-Alkoxy, Cyclohexyl oder Phenyl substituiertes Cyclohexyl, Cyclohexylmethyl oder Cyclohexyloxy, gegebenenfalls einfach bis dreifach, gleich oder verschieden durch Halogen, C₁-C₄-Alkyl oder C₁-C₄-Hälogenälkyl substituiertes Pyridyloxy, jeweils gegebenenfalls einfach bis vierfach, gleich oder verschieden durch Halogen, C₁-C₆-Alkyl, C₁-C₆-Halogenalkyl, C₁-C₆-Alkoxy_{,} C₁-C₆-Halogenalkoxy, C₁-C₆-Alkoxy-C₁-C₆-alkyl, C₁-C₆-Alkoxy-ethylenoxy, C₁-C₆-Alkylthio oder C₁-C₆-Halogenalkylthio substituiertes Phenyl, Benzyl, Benzoyl, Phenoxy, Phenylthio, Benzyloxy oder Benzylthio.

Die oben bei der Definition der erfindungsgemäßen Verbindungen genannten Kohlenwasserstoffreste, wie Alkyl sind - auch in Verbindung mit Heteroatomen, wie Alkoxy - soweit möglich, jeweils geradkettig oder verzweigt.
- Ar¹: steht besonders bevorzugt für Phenyl, das gegebenenfalls einfach bis dreifach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio,
- Ar²: steht besonders bevorzugt für Phenyl, das gegebenenfalls einfach bis vierfach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, n-, i-, s- oder t-Butyl, n-, i-, s- oder t-Pentyl, n-, i-, s- oder t-Hexyl, n- oder i-Octyl, n- oder i-Nonyl, n- oder i-Decyl, n- oder i-Dodecyl, Methoxy, Ethoxy, n- oder i-Propoxy, n-, i-, s- oder t-Butoxy, n-, i-, s- oder t-Pentyloxy, n-, i-, s- oder t-Hexyloxy, n- oder i-Octyloxy, n- oder i-Nonyloxy, n- oder i- Decyloxy, n- oder i-Dodecyloxy, Methylthio, Ethylthio, n- oder i-Propylthio, n-, i-, s- oder t-Butylthio, n-, i-, s- oder t-Pentylthio, n-, i-, s- oder t-Hexylthio, n- oder Octylthio, n- oder i-Nonylthio, n- oder i-Decylthio, n- oder i-Dodecylthio, Acetyl, Propionyl, n-oder i-Butyroyl, n-, i-, s- oder t-Valeroyl, Methoxycarbonyl, Ethoxycarbonyl, n- oder i-Propoxycarbonyl, n-, i-, s- oder t-Butoxycarbonyl, n-, i-, s- oder t-Pentoxycarbonyl, Methylendioxy, Ethylendioxy, jeweils gegebenenfalls durch Methyl, Ethyl, n- oder i-Propyl, n-, i-, s- oder t-Butyl, Methoxy, Ethoxy, n-oder i-Propoxy, n-, i-, s- oder t-Butoxy, Cyclohexyl oder Phenyl substituiertes Cyclohexyl, Cyclohexylmethyl oder Cyclohexyloxy, gegebenenfalls einfach bis dreifach gleich oder verschieden durch Fluor, Chlor, Brom, Methyl, Ethyl, n- oder i-Propyl, n-, i-, s- oder t-Butyl, oder durch Trifluormethyl substituiertes Pyridyloxy, jeweils gegebenenfalls einfach bis dreifach, gleich oder verschieden durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s-oder t-Butoxy, Difluormethoxy, Trifluormethoxy, Methyl-thio, Ethylthio, n-oder i-Propylthio, n-, i-, s- oder t-Butylthio, Difluormethylthio oder Trifluormethylthio substituiertes Phenyl, Benzyl, Benzoyl, Phenoxy, Phenylthio, Benzyloxy oder Benzylthio.
- Ar¹: steht ganz besonders bevorzugt für Phenyl, das gegebenenfalls einfach oder zweifach, gleich oder verschieden durch Fluor, Chlor, Brom oder Hydroxy, Methyl, Ethyl, n- oder i-Propyl, Methoxy, Ethoxy, n- oder i-Propoxy, Trifluormethyl oder Trifluormethoxy substituiert ist.
- Ar²: steht besonders bevorzugt für Phenyl, das gegebenenfalls einfach bis dreifach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, n-, i-, s- oder t-Butyl, n-, i-, s- oder t-Pentyl, n-, i-, s- oder t-Hexyl, n- oder i-Octyl, n- oder i-Nonyl, n- oder i-Decyl, n- oder i-Dodecyl, Methoxy, Ethoxy, n- oder i-Propoxy, n-, i-, s- oder t-Butoxy, n-, i-, s- oder t-Pentyloxy, n-, i-, s- oder t-Hexyloxy, n- oder i-Octyloxy, n- oder i-Nonyloxy, n- oder Decyloxy, n- oder i-Dodecyloxy, Methylthio, Ethylthio, n- oder i-Propylthio, n-, i-, s- oder t-Butylthio, n-, i-, s- oder t-Pentylthio, n-, i-, s- oder t-Hexylthio, n- oder Octylthio, n- oder i-Nonylthio, n- oder i-Decylthio, n- oder i-Dodecylthio, Acetyl, Propionyl, n-oder i-Butyroyl, n-, i-, s- oder t-Valeroyl, Methoxycarbonyl, Ethoxycarbonyl, n- oder i-Propoxycarbonyl, n-, i-, s- oder t-Butoxycarbonyl, n-, i-, s- oder t-Pentoxycarbonyl, Methylendioxy, Ethylendioxy, jeweils gegebenenfalls durch Methyl, Ethyl, n- oder i-Propyl, n-, i-, s- oder t-Butyl, Methoxy, Ethoxy, n-oder i-Propoxy, n-, i-, s- oder t-Butoxy, Cyclohexyl oder Phenyl substituiertes Cyclohexyl, Cyclohexylmethyl oder Cyclohexyloxy, gegebenenfalls einfach bis dreifach gleich oder verschieden durch Fluor, Chlor, Brom, Methyl, Ethyl, n- oder i-Propyl, n-, i-, s- oder t-Butyl, oder durch Trifluormethyl substituiertes Pyridyloxy, jeweils gegebenenfalls einfach bis dreifach, gleich oder verschieden durch Fluor, Chlor, Brom, Methyl, Ethyl, n- oder i-Propyl, n-, i-, s- oder t-Butyl, Trifluormethyl, Methoxy, Ethoxy, n- oder i-Propoxy, n-, i-, s-oder t-Butoxy, Difluormethoxy, Trifluormethoxy, Methyl-thio, Ethylthio, n-oder i-Propylthio, n-, i-, s- oder t-Butylthio, Difluormethylthio oder Trifluormethylthio substituiertes Phenyl, Benzyl, Benzoyl, Phenoxy, Phenylthio, Benzyloxy oder Benzylthio.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte, für die Isomerengemische sowie den reinen E- oder Z-Isomeren.

Diese Restedefinitionen können untereinander, also auch zwischen den angegebenen Bereichen bevorzugter Verbindungen, beliebig kombiniert werden.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Beispielhaft seien für die Gruppierung Ar' in der Formel (I) folgende Bedeutungen genannt:

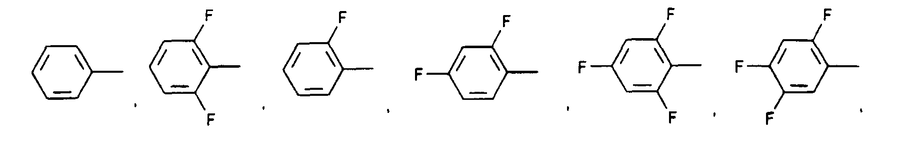

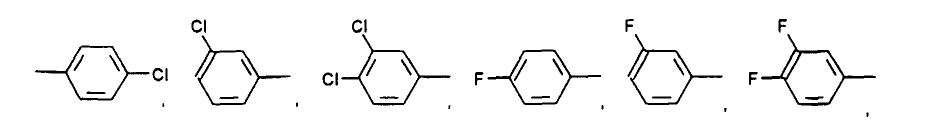

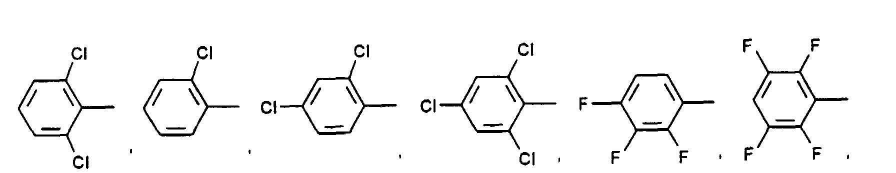

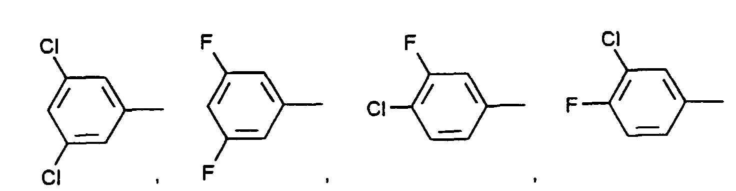

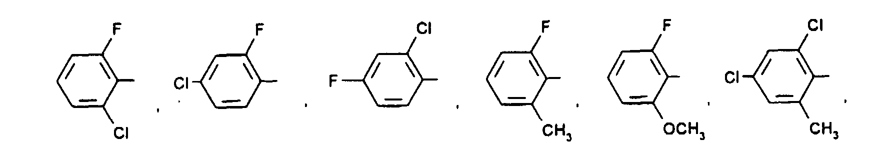

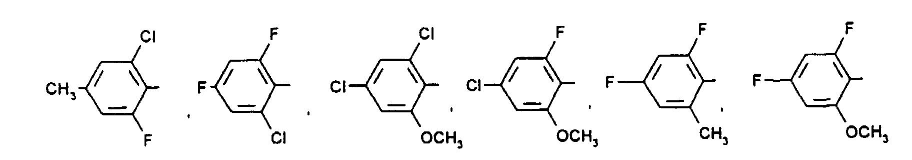

Beispielhaft seien für die Gruppierung Ar² in der Formel (I) folgende Bedeutungen genannt: 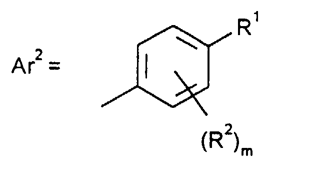
- R¹ =: gemäß Tabelle 1
- (R²)ₘ =: gemäß Tabelle 2

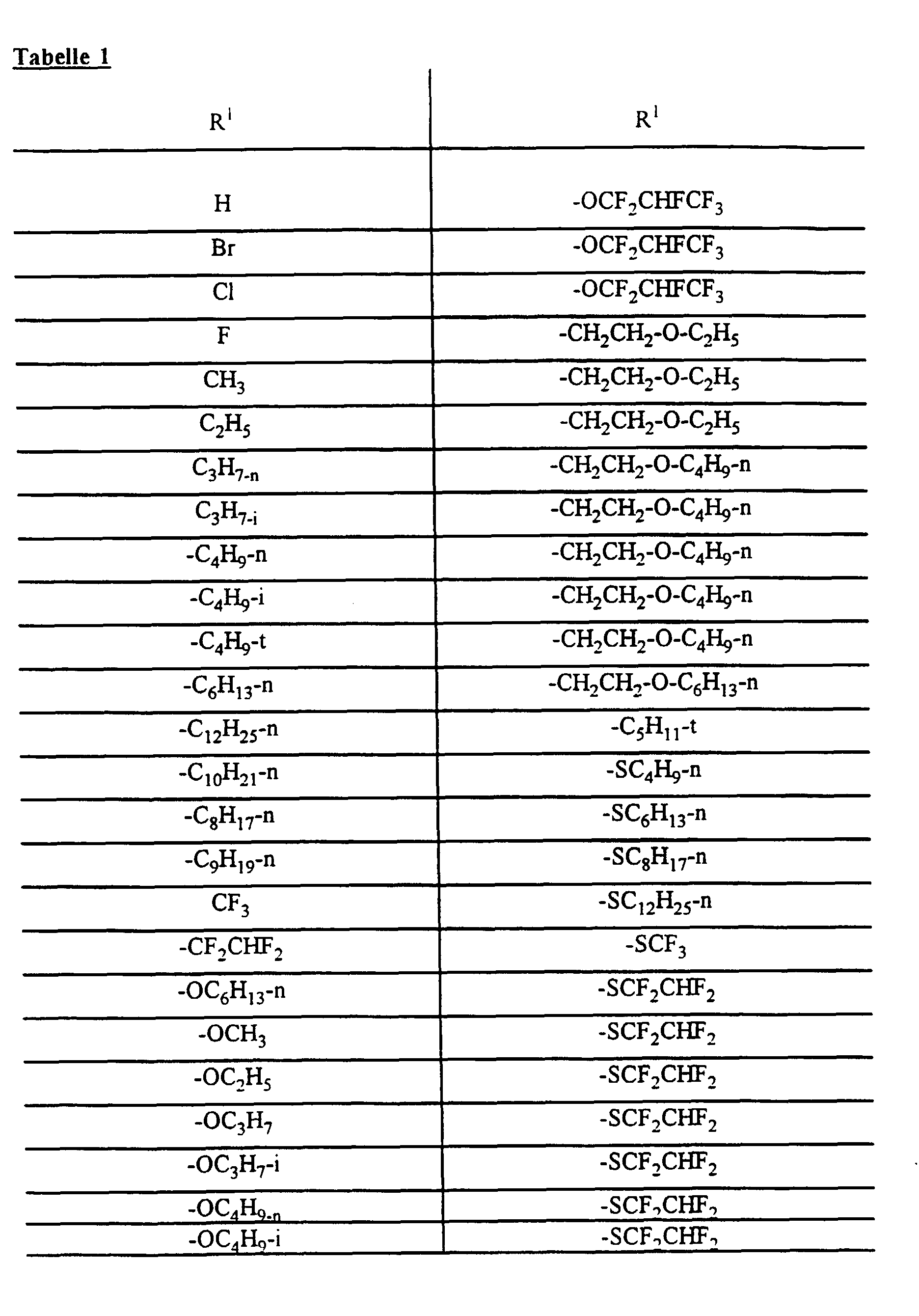

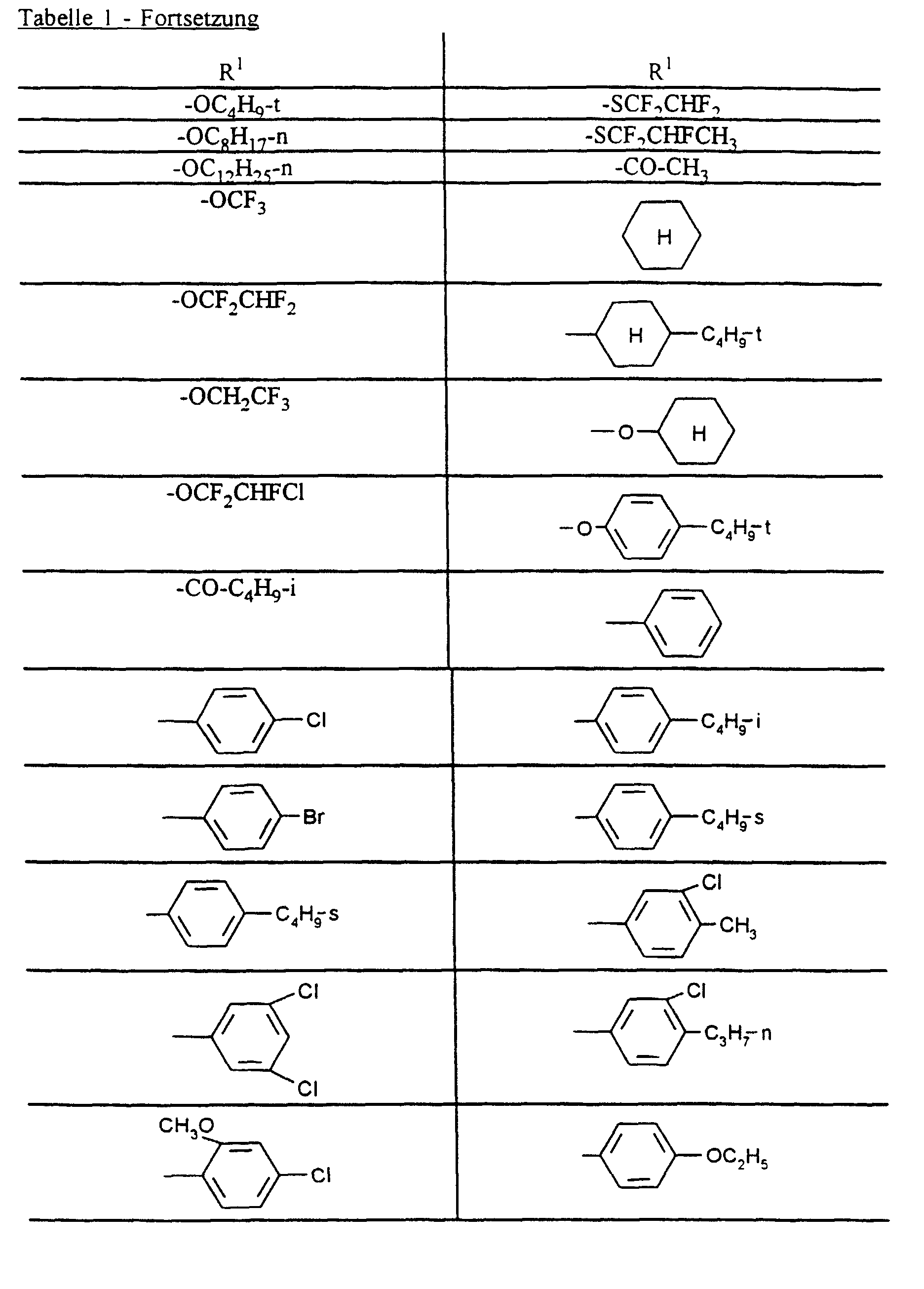

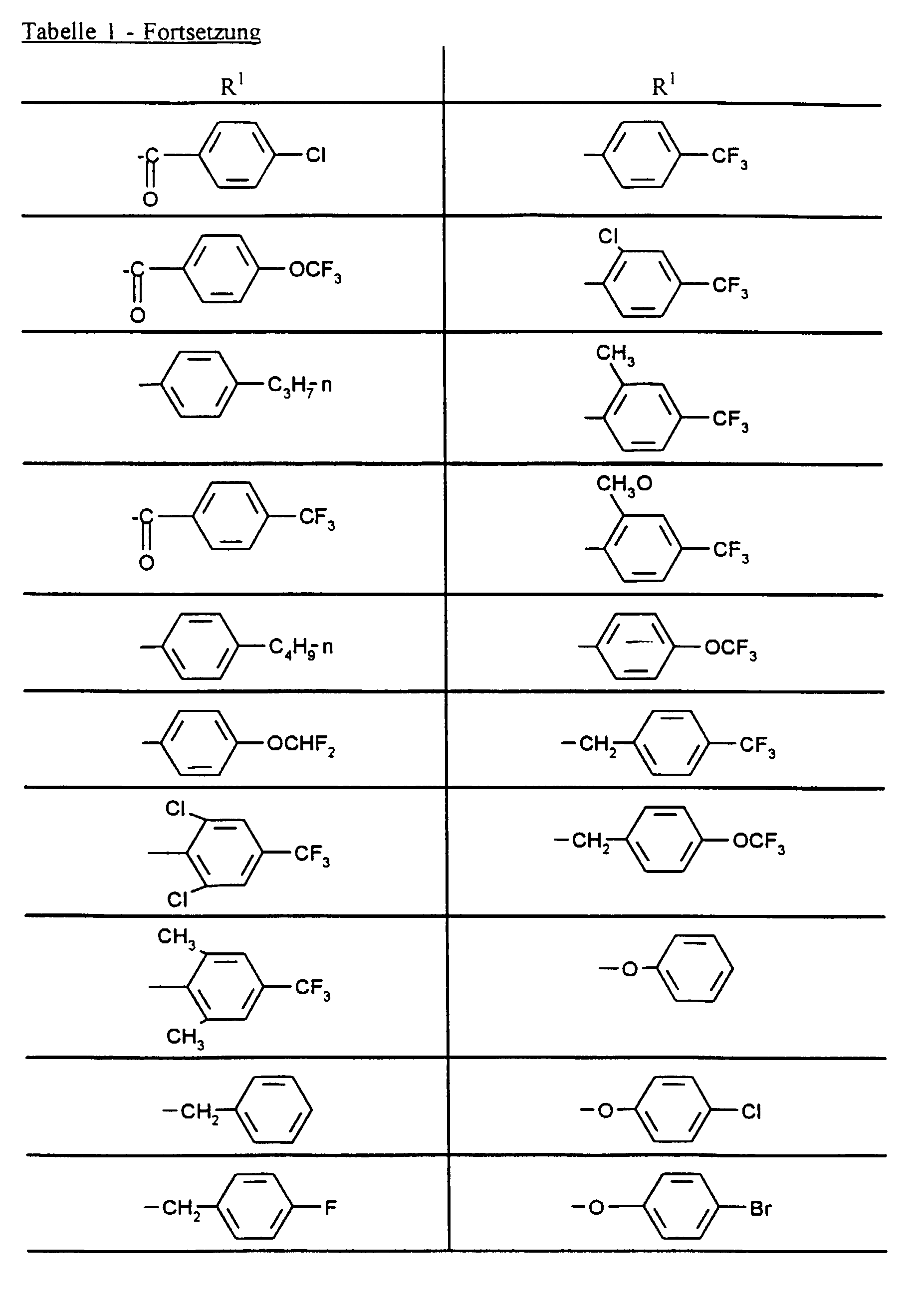

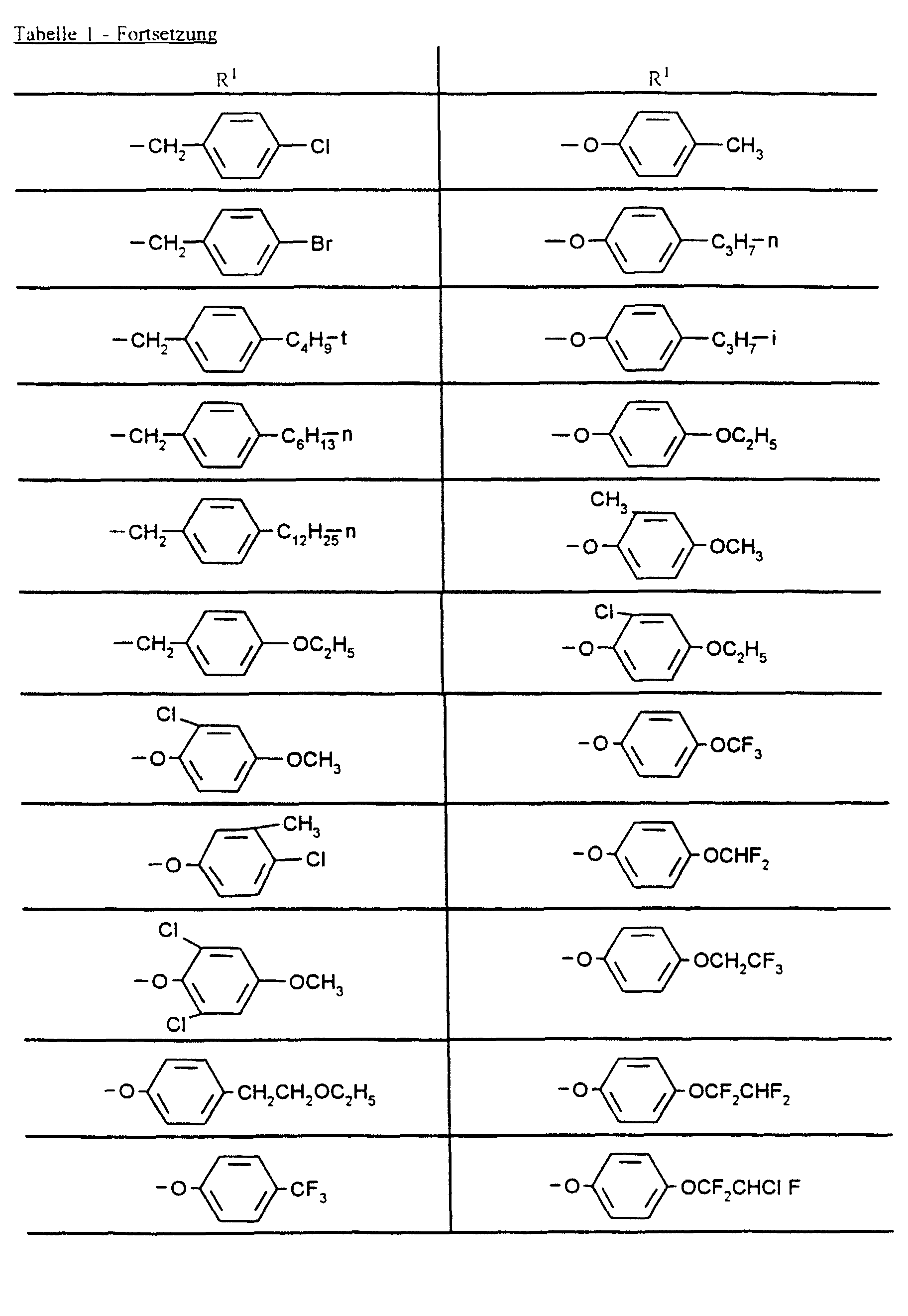

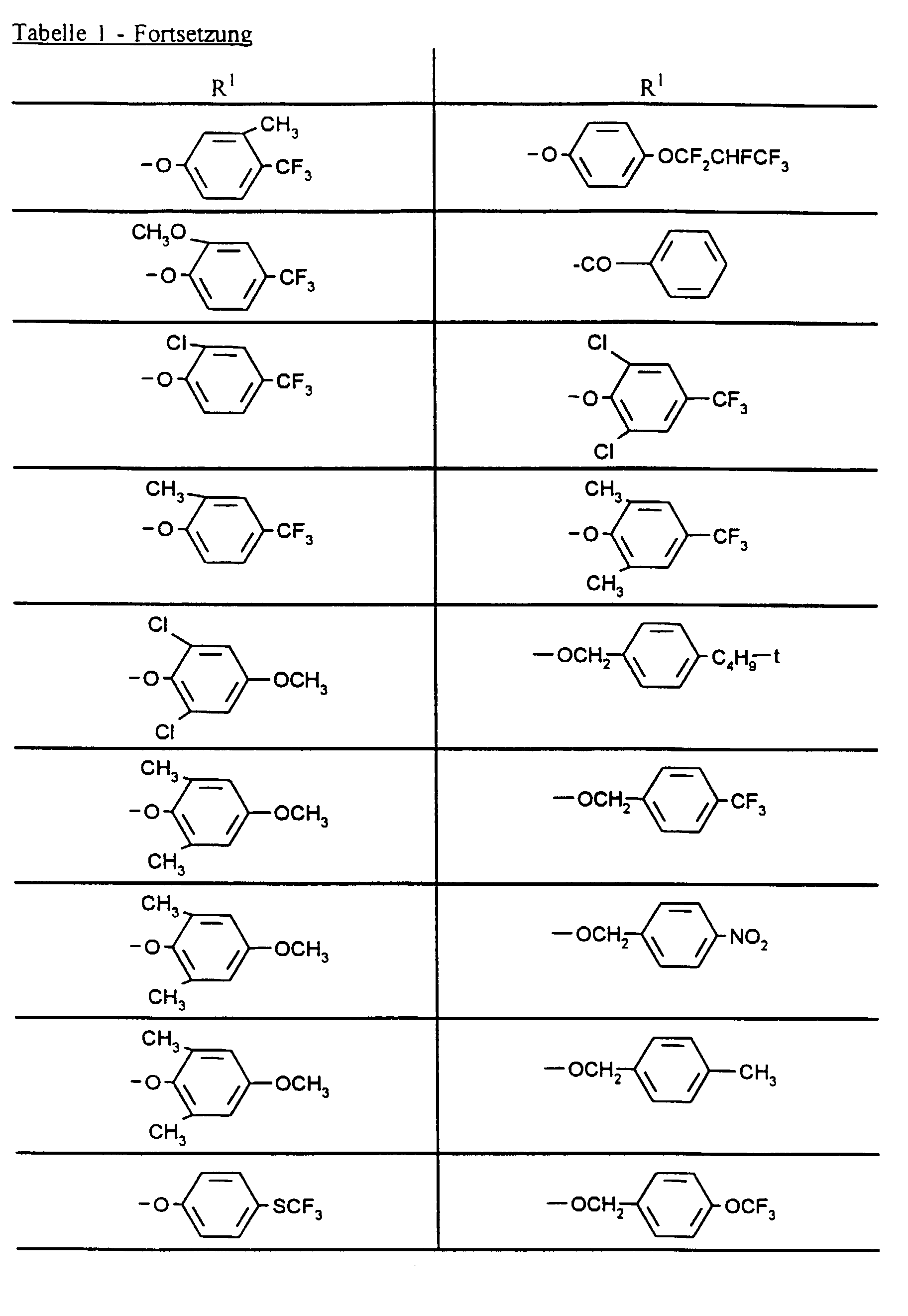

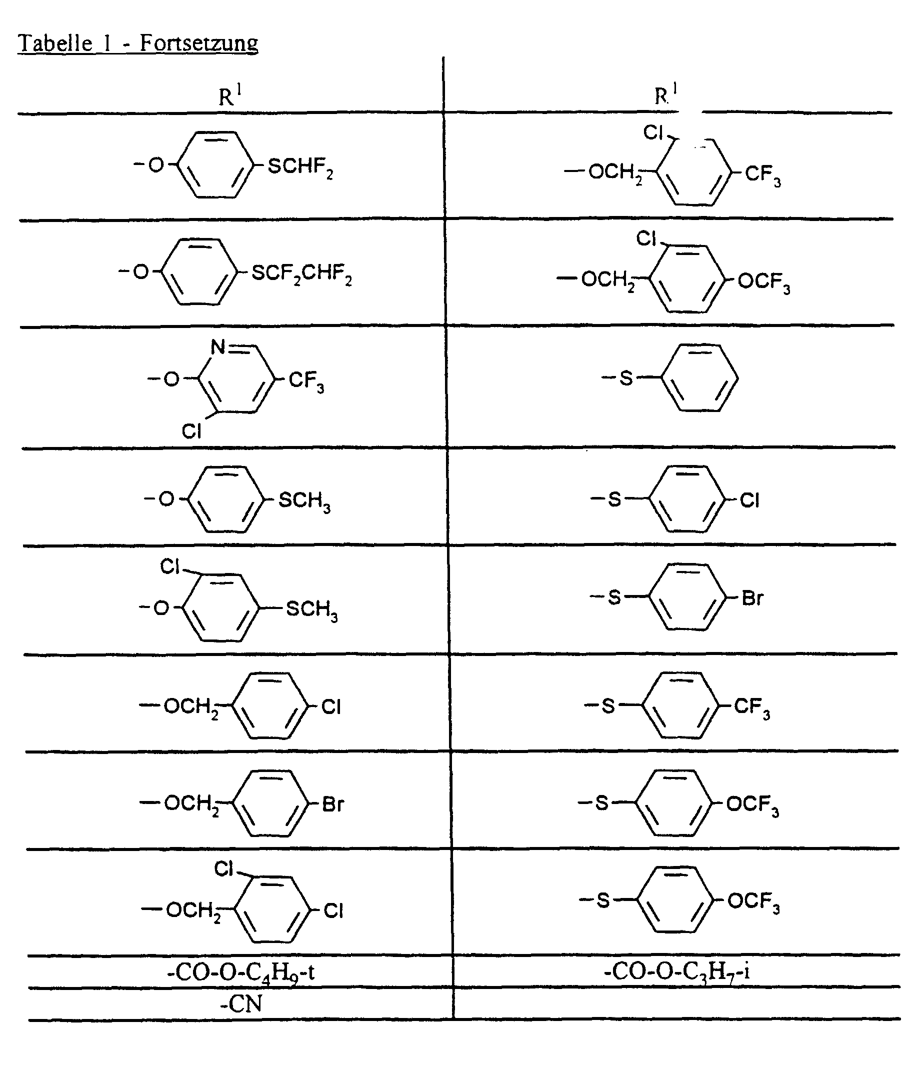

**Tabelle 2**

| (R²)ₘ | (R²)ₘ |
|---|---|
| H | 2-CH₃ |
| | 3,5-(CH₃)₂ |
| | 2,6-(CH₃)₂ |
| 2-Cl | 2-OCH₃ |
| 2-F | 3-OCH₃ |
| 3-Cl | 2,6-OC₂H₅ |
| 2,6-Cl₂ | 3-CH₃ |
| 3,5-Cl₂ | 3,5-OCH₃ |
| 3,5-F₂ | 3-OC₆H₅ |
| 2,5-Cl₂ | 2-Cl; 5-CF₃ |
| 3,5-Cl₂; 2-F | 2-Cl; 3-CF₃ |
| 2,3-F₂ | |
| 2,5-F₂ | gemeinsam mit R¹ für |
| 2,3,5,6-Cl₄ | 3,4-OCF₂O- |
| 3-CF₃ | 3,4-OCF₂CF₂O |

In der nachstehenden Tabelle 3 sind Beispiele für die erfindungsgemäßen Verbindungen der Formel (Ia) aufgeführt:

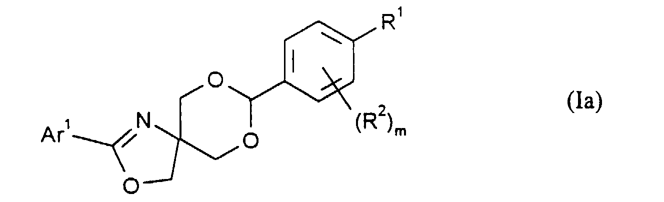

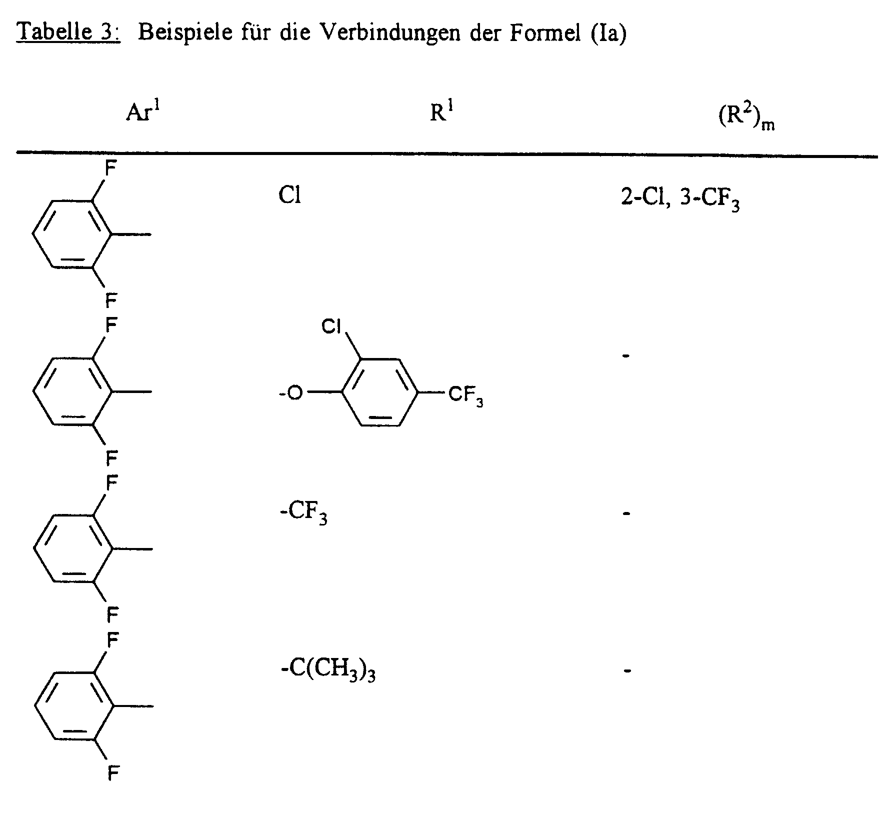

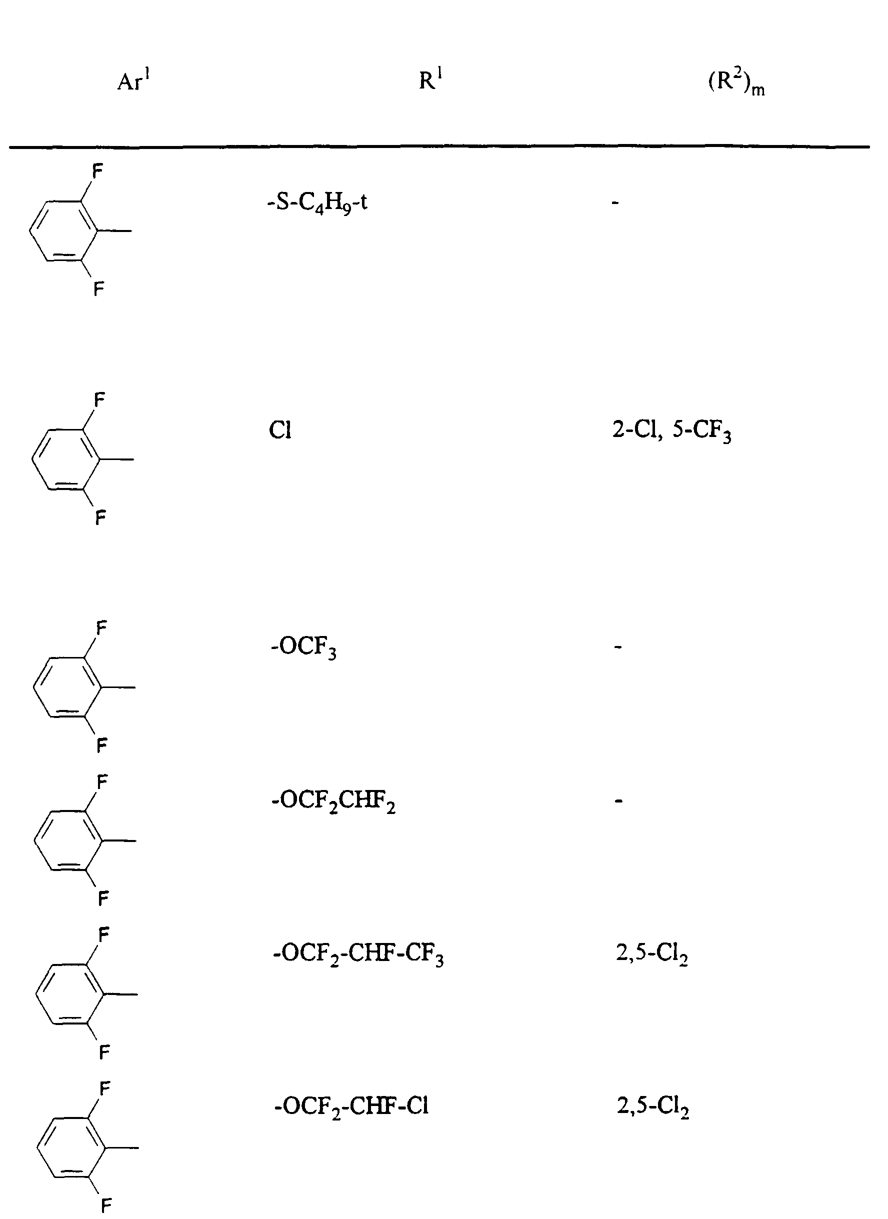

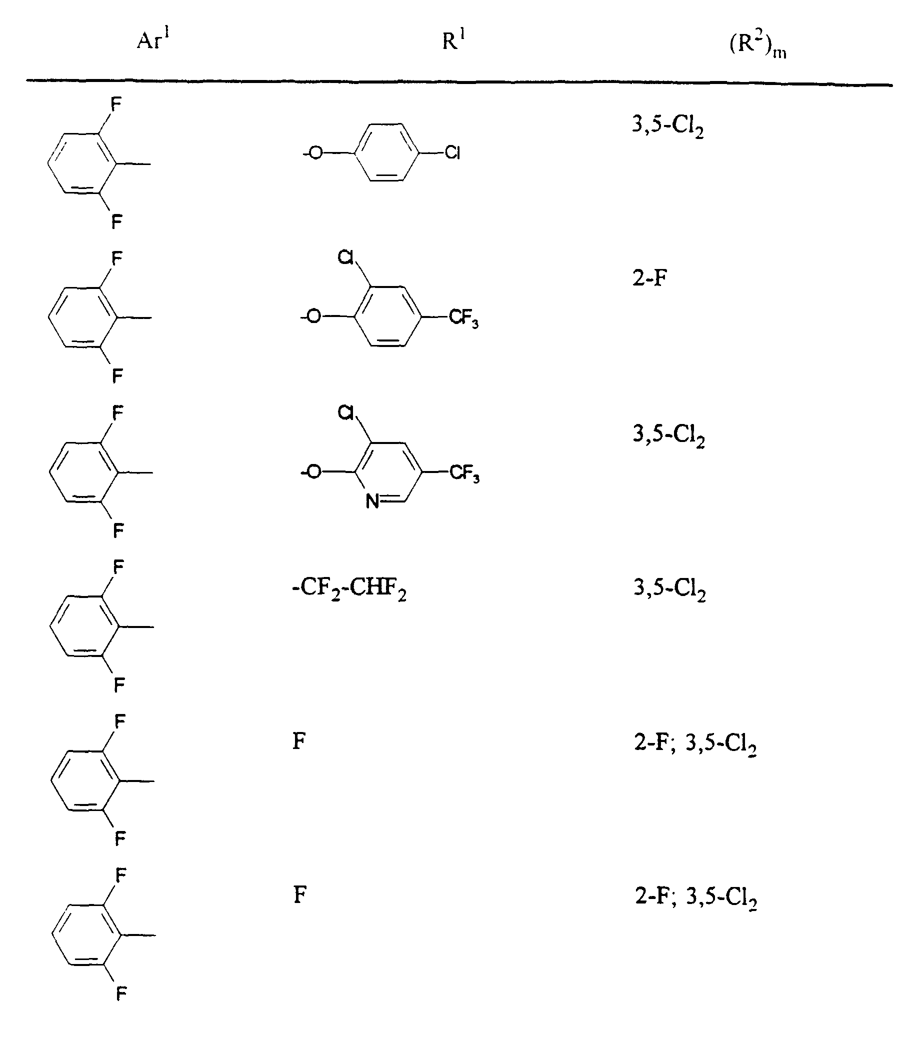

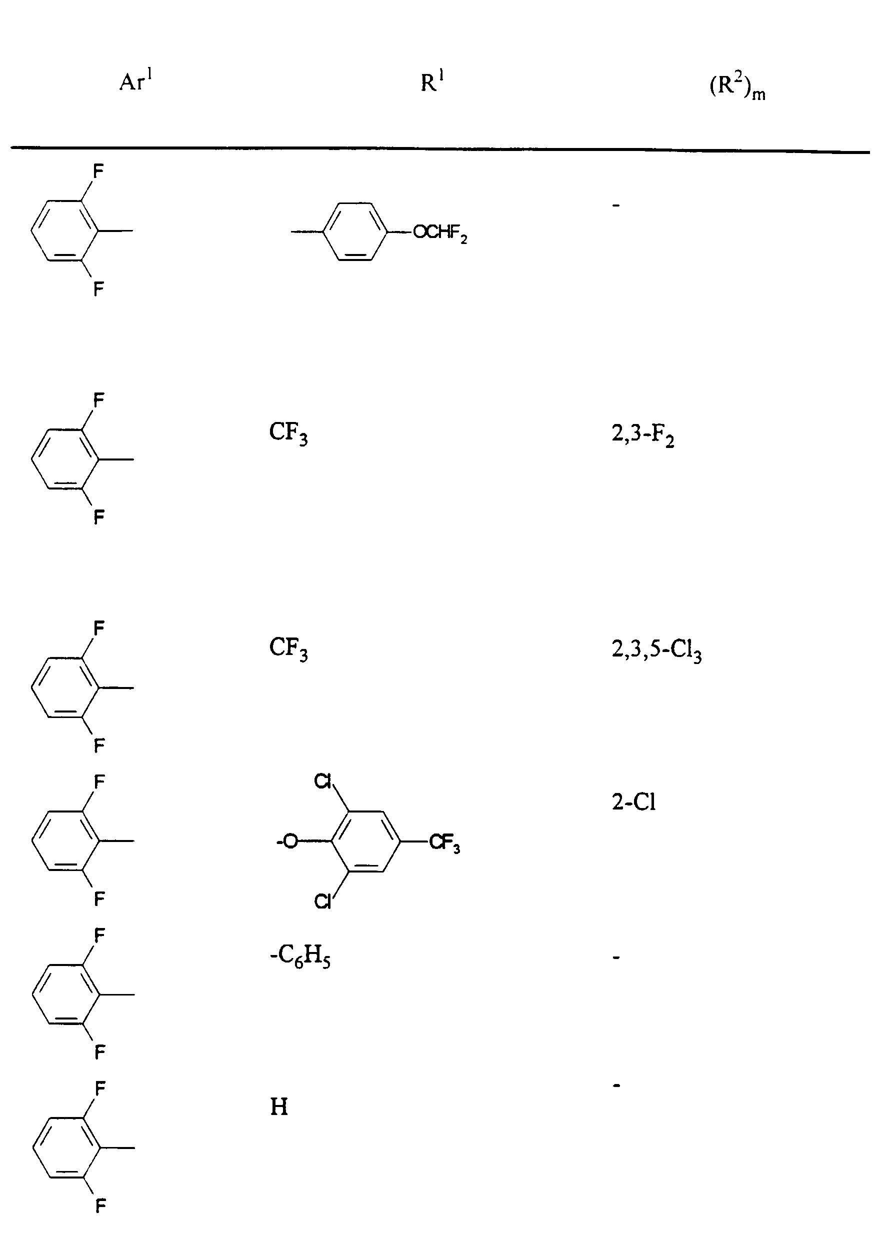

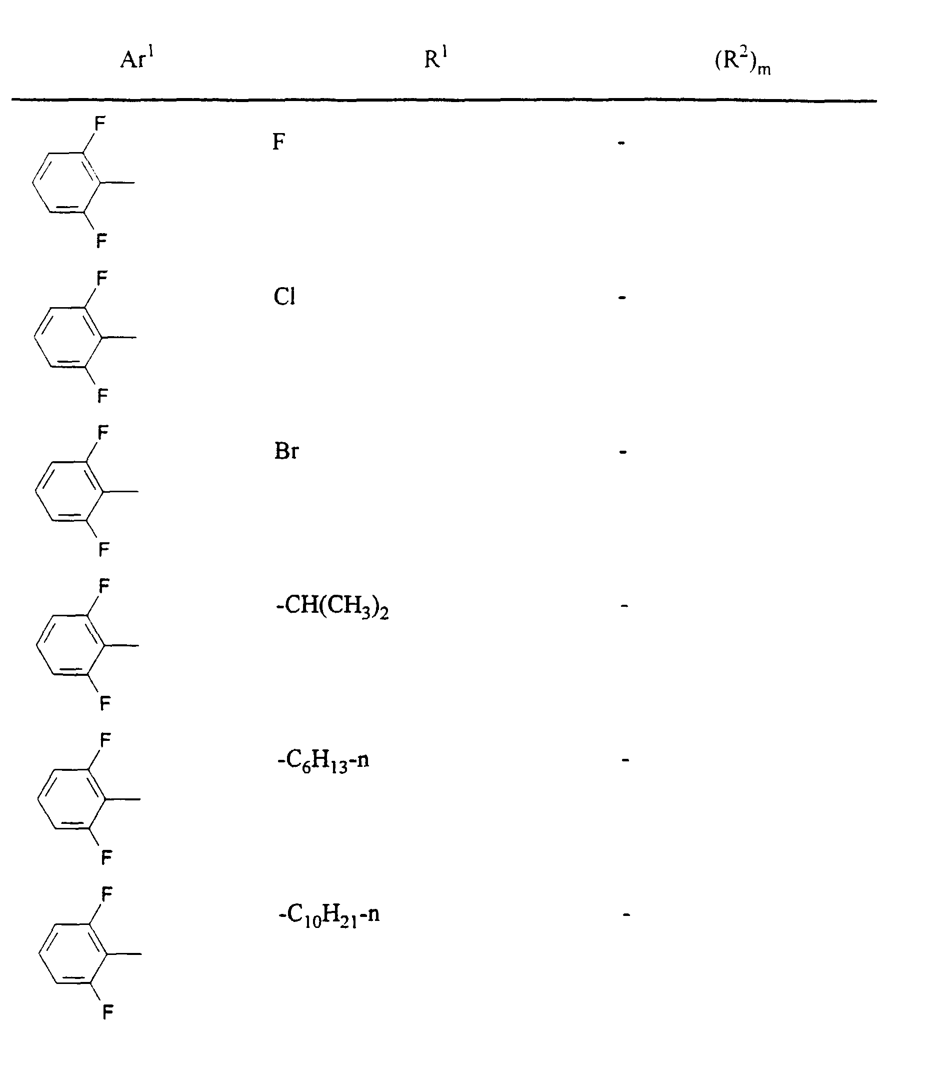

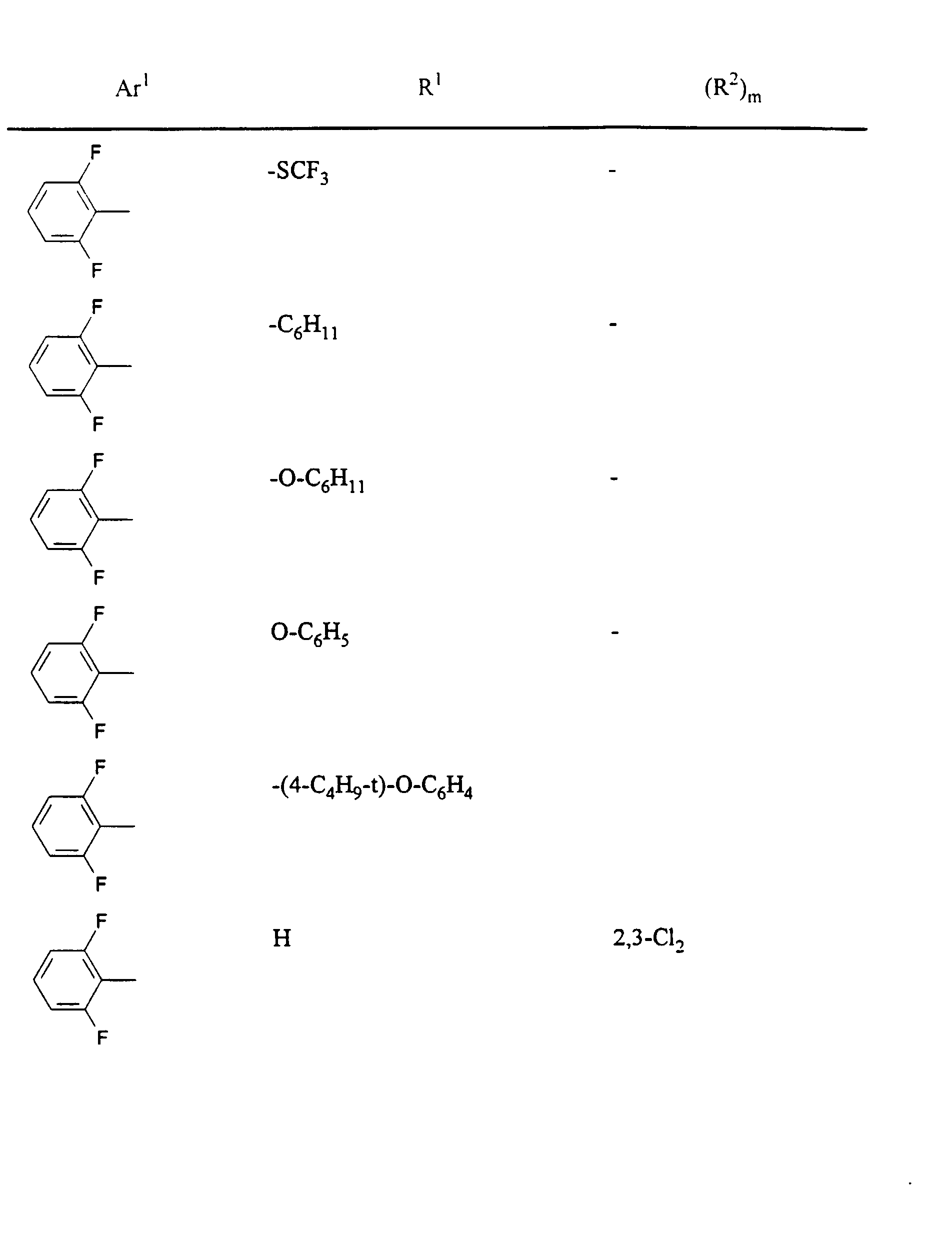

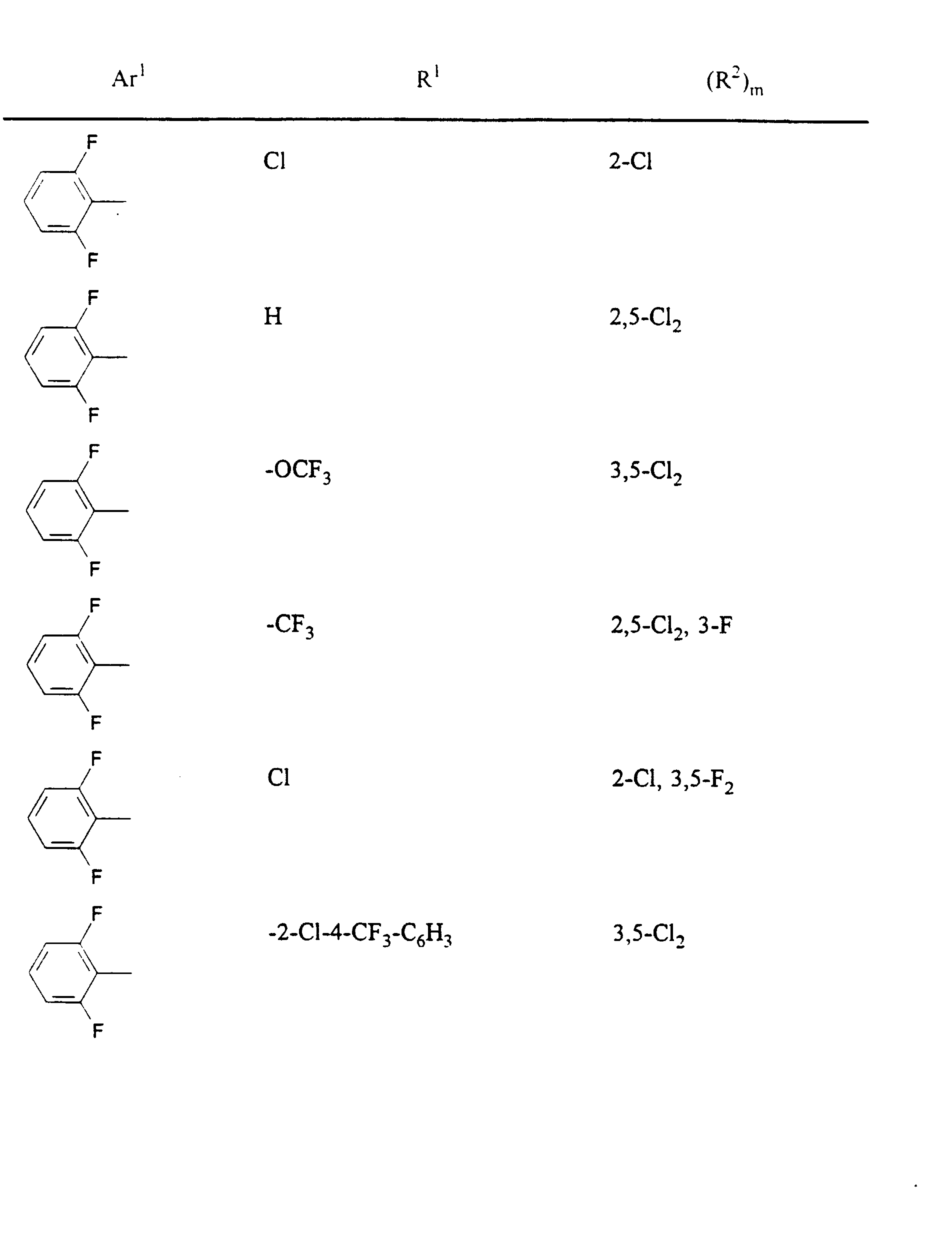

### Tabelle 4

Die Verbindungen der Tabelle 4 entsprechen den in Tabelle 3 aufgeführten Verbindungen der Formel (Ia), wobei Ar¹ jedoch für 2-Chlor-6-fluor-phenyl steht und m, R¹ und R² die in Tabelle 3 angegebenen Bedeutungen haben.

### Tabelle 5

Die Verbindungen der Tabelle 5 entsprechen den in Tabelle 3 aufgeführten Verbindungen der Formel (Ia), wobei Ar¹ jedoch für 2,6-Dichlor-phenyl steht und m, R¹ und R² die in Tabelle 3 angegebenen Bedeutungen haben.

### Tabelle 6

Die Verbindungen der Tabelle 6 entsprechen den in Tabelle 3 aufgeführten Verbindungen der Formel (Ia), wobei Ar¹ jedoch für 2-Chlor-phenyl steht und m, R¹ und R² die in Tabelle 3 angegebenen Bedeutungen haben.

### Tabelle 7

Die Verbindungen der Tabelle 7 entsprechen den in Tabelle 3 aufgeführten Verbindungen der Formel (Ia), wobei Ar¹ jedoch für 2-Fluor-phenyl steht und m, R¹ und R² die in Tabelle 3 angegebenen Bedeutungen haben.

Verwendet man zu Durchführung des erfindungsgemäßen Verfahrens (a) beispielsweise5-Amino-5-hydroxymethyl-2-(4-methylphenyl )-1,3-dioxanund2-Chlor-benzoesäure als Ausgangsstoffe und Polyphosphorsäure (PPS) als wasser-entziehendes Mittel, so kann der Reaktionsablauf durch das folgende Formelschema skizziert werden: 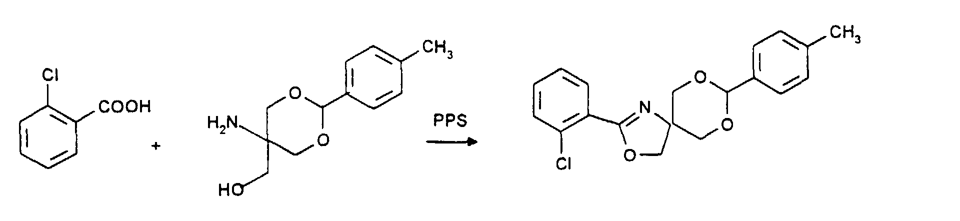 Verwendet man zur Durchführung des erfindungsgemäßen Verfahrens (b) beispielsweise 5-Hydroxymethyl-5-(2-fluor-benzoylamino)-2-(4-methoxy-phenyl)-1,3-dioxan als Ausgangsverbindung und Polyphosphorsäure (PPS) als wasser-entziehendes Mittel, so kann der Reaktionsablauf durch das folgende Formelschema skizziert werden: 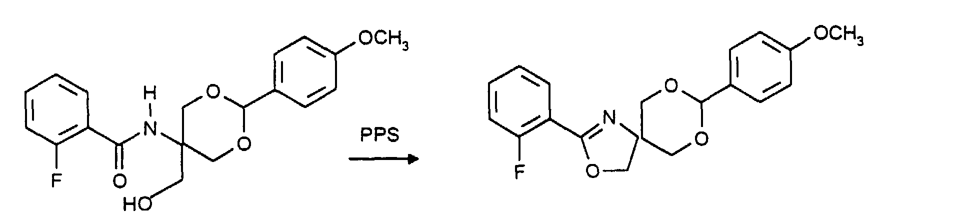 Verwendet man zur Durchführung des erfindungsgemäßen Verfahrens (c) beispielsweise 5-Chlormethyl-5-(2-fluor-benzoylamino)-2-(4-trifluormethyl)-1,3-dioxanals Ausgangsverbindung und Triethylamin als Base, so kann der Reaktionsablauf durch das folgende Formelschema skizziert werden: 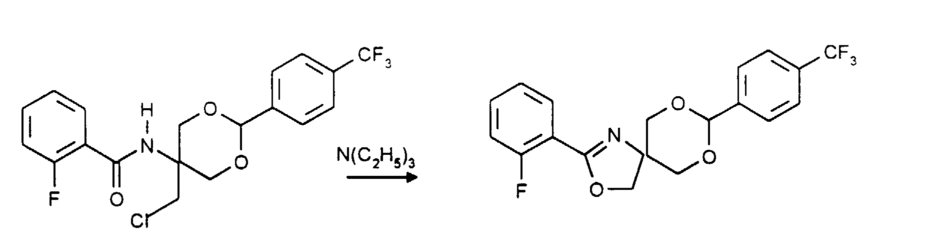 Verwendet man zur Durchführung des erfindungsgemäßen Verfahrens (d) beispielsweise 5-(2,4-Dichlorbenzoyloxymethyl)-5-(2,4-dichlorbenzoylamino)-2-(4-chlorphenyl)-1,3-dioxan als Ausgangsverbindungen und Kalium-tert.-butylat als Base, so kann der Reaktionsverlauf durch das folgende Schema dargestellt werden:

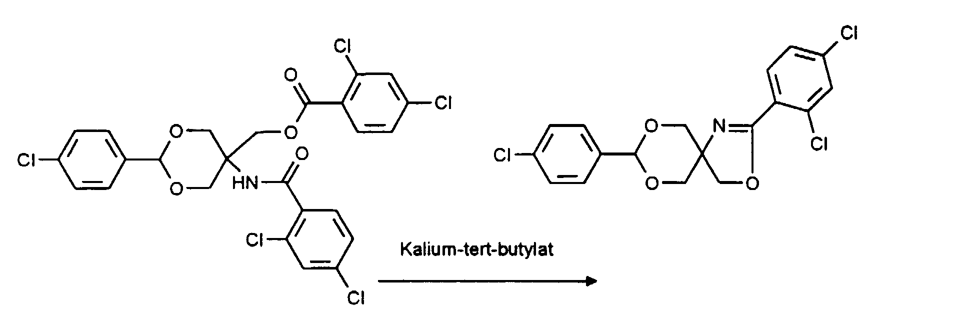

Die beim erfindungsgemäßen Verfahren zur Herstellung der Verbindungen der allgemeinen Formel (I) als Ausgangsstoffe zu verwendenden Aminoalkohole sind durch die Formel (II) allgemein definiert. In der Formel (II) hat Ar² vorzugsweise bzw. insbesondere diejenige Bedeutung, die bereits oben im Zusammenhang mit der Beschreibung der Verbindungen der Formel (I) als bevorzugt bzw. als insbesondere bevorzugt für Ar² angegeben wurde.

Die Aminoalkohole der Formel (II) sind mit Ausnahme der Verbindung der Formel (II), in welcher Ar² für Phenyl steht, noch nicht aus der Literatur bekannt; sie sind als neue Stoffe Gegenstand der vorliegenden Anmeldung.

Man erhält die neuen Aminoalkohole der Formel (II), wenn man Nitroalkohole der Formel (VII) 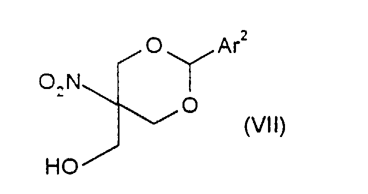 in welcher
- Ar²: die oben angegebene Bedeutung hat,
mit Wasserstoff in Gegenwart eines Katalysators, wie z.B. Raney-Cobalt oder Raney-Nickel, Palladium oder Platin (z.B. auf Kohle als Trägermaterial), gegebenenfalls in Gegenwart eines Verdünnungsmittels, wie z.B. Methanol oder Ethanol, bei Temperaturen zwischen -20°C und +200C, vorzugsweise zwischen 0°C und 150°C, und bei Drücken zwischen 1 bar und 300 bar, vorzugsweise zwischen 10 bar und 200 bar, umsetzt (vgl. die Herstellungsbeispiele).

Die als Vorprodukte benötigten Nitroalkohole der Formel (VII) sind teilweise bekannt und/oder können nach an sich bekannten Verfahren hergestellt werden (vgl. J. Am. Chem. Soc. 63 (1941), 2635-2636; Synthesis 1993, 815-818, Herstellungsbeispiele).

Die Nitroalkohole der Formel (VII) sind mit Ausnahme derjenigen Verbindungen, bei denen Ar² für Phenyl, 4-Methoxy-phenyl, 4-Cyano-phenyl, 2-Nitro-phenyl, 3-Nitrophenyl,4-Nitro-phenyl, 4-Chlor-phenyl, 4-Dimethylamino-phenyl, 4-Hydroxy-phenyl, 2-Hydroxy-phenyl, 3-Ethoxy-4-hydroxy-phenyl oder 4-Hydroxy-3-methoxy-phenyl steht, noch nicht aus der Literatur bekannt und sind mit Ausnahme der vorgenannten Verbindungen als neue Stoffe Gegenstand der vorliegenden Anmeldung.

Man erhält die Nitroalkohole der Formel (VII), wenn man Aldehyde der Formel (VIII)

Ar²-CHO (VIII)

in welcher
- Ar²: die oben angegebene Bedeutung hat,
mit Tris-hydroxymethyl-nitromethan der Formel (IX)

(HOCH₂)₃C-NO₂ (IX)

in Gegenwart eines zur azeotropen Wasserabscheidung geeigneten Lösungsmittels, wie z.B. Toluol, und in Gegenwart eines Katalysators, wie z.B. p-Toluolsulfonsäure, an einem Wasserabscheider bei Temperaturen zwischen 50°C und 150°C umsetzt (vgl. die Herstellungsbeispiele) oder wenn man Verbindungen der Formel (VII) mit Trihydroxymethylnitromethan der Formel (VIII) in Gegenwart von Säuren oder Lewis-Säuren und einem wasserentziehenden Reagenz, z.B. Orthoameisensäuretrimethylester bei -20°C bis 120°C umsetzt.

Die beim erfindungsgemäßen Verfahren (a) zur Herstellung der Verbindungen der Formel (I) weiter als Ausgangsstoffe zu verwendenden Carbonsäuren sind durch die Formel (III) allgemein definiert. In der Formel (III) hat Ar¹ vorzugsweise bzw. insbesondere diejenige Bedeutung, die bereits oben im Zusammenhang mit der Beschreibung der Verbindungen der Formel (I) als bevorzugt bzw. als insbesondere bevorzugt für Ar¹ angegeben wurde.

Die Ausgangsstoffe der Formel (III) sind bekannte organischen Synthesechemikalien.

Die erfindungsgemäßen Verfahren (a) und (b) werden unter Verwendung eines wasser-entziehenden Mittels durchgeführt. Es können die in der organischen Chemie üblichen wasser-entziehenden Mittel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid, Natriumsulfat, Calciumchlorid, Magnesiumsulfat, Orthoameisensäuretrimethylester oder -triethylester, Molekularsiebe und das System Triphenylphosphin/Triethylamin/Tetrachlormethan.

Als Verdünnungsmittel zur Durchführung der erfindungsgemäßen Verfahren (a) bis (c) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Tetralin, Dichlormethan, Chloroform, Tetrachlormethan; Ether, wie t-Butyl-methylether, Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol, Ethanol, n- oder i-Propanol. Weiterhin organische basische Lösungsmittel wie z.B. Pyridin, Picoline, Lutidine oder Collidin.

Die Reaktionstemperaturen können bei der Durchführung des erfindungsgemäßen Verfahrens (a) in einem größeren Bereich variiert werden. Im allgemeinen arbeitet man bei Temperaturen zwischen -20°C und +200°C, vorzugsweise bei Temperaturen zwischen 0°C und 150°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasser-entziehenden Mittels durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden.

In einer besonderen Ausführungsform des erfindungsgemäßen Verfahrens (a) können statt der Carbonsäuren der Formel (III) auch entsprechende Nitrile eingesetzt werden, wobei dann vorzugsweise an Stelle eines wasser-entziehenden Mittels ein Katalysator, wie z.B. Zink-(II)-chlorid verwendet wird.

Die beim erfindungsgemäßen Verfahren (b) zur Herstellung der Verbindungen der allgemeinen Formel (I) als Ausgangsstoffe zu verwendenden Amidalkohole sind durch die Formel (IV) allgemein definiert. In der Formel (IV) haben Ar¹ und Ar² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Ar¹ und Ar² angegeben wurden.

Die Amidalkohole der Formel (IV) sind noch nicht aus der Literatur bekannt; sie sind als neue Stoffe Gegenstand der vorliegenden Anmeldung.

Man erhält die neuen Amidalkohole der Formel (IV), wenn man Aminoalkohole der Formel (II) - oben - mit angenähert äquimolaren Mengen von Carbonsäurehalogeniden der Formel (X)

Ar¹-CO-X¹ (X)

in welcher
- Ar¹: die oben angegebene Bedeutung hat und
- X¹: für Halogen (vorzugsweise Fluor, Chlor oder Brom, insbesondere Chlor) steht,
gegebenenfalls in Gegenwart eines Säureakzeptors, wie z.B. Pyridin, Triethylamin oder Kaliumcarbonat, und gegebenenfalls in Gegenwart eines Verdünnungsmittels, wie z.B. Toluol, Tetrahydrofuran, Aceton, Methyl-isobutylketon oder Acetonitril, bei Temperaturen zwischen -80°C und +200°C, vorzugsweise zwischen -30°C und +120°C umsetzt.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) zur Herstellung der Verbindungen der Formel (I) setzt man pro Mol an Amidalkohol der Formel (IV) im allgemeinen 1 bis 20 Mol, vorzugsweise 1 bis 5 Mol an wasser-entziehendem Mittel ein.

In einer bevorzugten Ausführungsform des erfindungsgemäßen Verfahrens (b) wird der Amidalkohol der Formel (IV) in einem Verdünnungsmittel vorgelegt und das wasser-entziehende Mittel wird dann eindosiert. Das Reaktionsgemisch wird bei der erforderlichen Temperatur bis zum Ende der Umsetzung gerührt und anschließend auf übliche Weise aufgearbeitet.

Die beim erfindungsgemäßen Verfahren (c) zur Herstellung der Verbindungen der allgemeinen Formel (I) als Ausgangsstoffe zu verwendenden Carbonsäureamide sind durch die Formel (V) allgemein definiert. In der Formel (V) haben Ar¹ und Ar² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Ar¹ und Ar² angegeben wurden; X steht vorzugsweise für Chlor, Brom, Methylsulfonyloxy, Phenylsulfonyloxy oder Tolylsulfonyloxy, insbesondere für Chlor und Methylsulfonyl.

Die Carbonsäureamide der Formel (V) sind noch nicht aus der Literatur bekannt; sie sind als neue Stoffe Gegenstand der vorliegenden Anmeldung.

Man erhält die Carbonsäureamide der Formel (V), wenn man entsprechende Amidalkohole der Formel (IV) mit Chlorierungsmitteln, wie z.B. Phosgen, Thionylchlorid, Phosphor(III)-bromid oder Phosphor(V)-chlorid, bzw. mit Sulfonylierungsmitteln, wie z.B. Methansulfonsäurechlorid, Benzolsulfonsäurechlorid oder p-Toluolsulfonsäurechlorid, gegebenenfalls in Gegenwart eines Verdünnungsmittels, wie z.B. Methylenchlorid, Tetrahydrofuran, Toluol, Chlorbenzol, Pyridin oder Tetrachlormethan, bei Temperaturen zwischen -20°C und 150°C umsetzt.

Das erfindungsgemäße Verfahren (c) wird in Gegenwart einer Base durchgeführt. Es kommen hierbei alle üblichen anorganischen oder organischen Basen in Betracht. Vorzugsweise verwendbar sind Erdalkali- oder Alkalimetall-hydride, -oxide, - hydroxide, -amide, -alkoholate, -acetate, -carbonate oder hydrogencarbonate, wie beispielsweise Natriumhydrid, Calciumhydrid, Calciumoxid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, Picolin, Lutidin, Collidin, N-Methylpiperidin, N,N-Dimethylamino-pyridin, Diazabicyclooctan (DABCO), Diazabicyclononen (DBN) oder Diazabicycloundecen (DBU). Es ist jedoch auch möglich die tertiären Amine als Lösungsmittel einzusetzen.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +200°C, vorzugsweise bei Temperaturen zwischen 10°C und 150°C.

Das erfindungsgemäße Verfahren (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (c) zur Herstellung der Verbindungen der Formel (I) setzt man pro Mol an Carbonsäureamid der Formel (V) im allgemeinen 1 bis 3 Mol, vorzugsweise 1,0 bis 1,5 Mol einer Base ein.

In einer bevorzugten Ausführungsform des erfindungsgemäßen Verfahrens (c) wird das Carbonsäureamid der Formel (V) zunächst aus einem Amidalkohol der Formel (IV) wie oben angegeben erzeugt (vgl. auch die Herstellungsbeispiele) und nach Einengen unter vermindertem Druck wird das als Rückstand erhaltene Rohprodukt der Formel (V) mit einer Base in einem geeigneten Verdünnungsmittel vermischt; das Gemisch wird bei der erforderlichen Temperatur bis zum Ende der Umsetzung gerührt und anschließend auf übliche Weise aufgearbeitet (vgl die Herstellungsbeispiele).

In einer weiteren bevorzugten Ausführungsform des erfindungsgemäßen Verfahrens (c) wird in einem basischen Lösungsmittel insbesondere Pyridin zunächst das Carbonsäureamid der Formel (V), wie oben angegeben, erzeugt und anschließend bei erhöhter Temperatur die Verbindungen der Formel (I) generiert.

Die Carboxamidbenzoate der Formel (VI), die beim erfindungsgemäßen Verfahren (d) zur Herstellung der Verbindungen der allgemeinen Formel (I) verwendet werden, sind noch nicht in der Literatur bekannt und sind als neue Stoffe Gegenstand der vorliegenden Anmeldung. In der Formel (VI) haben Ar¹ und Ar² vorzugsweise bzw. insbesondere diejenigen Bedeutungen die bereits oben im Zusammenhang mit der Beschreibung der Verbindungden der Formel (I) als bevorzugt bzw. als insbesondere bevorzugt für Ar¹ und Ar² angegeben sind.

Man erhält die Carboxamidderivate der Formel (VI) beispielsweise, wenn man Aminoalkohole der Formel (II) mit einer etwa doppeltäquimolaren Menge an Carbonsäurehalogenid der Formel (X) gegebenenfalls in Gegenwart eines Säureakzeptors wie z.B. Pyridin, Triethylamin oder Kaliumcarbonat und gegebenenfalls in Gegenwart eines Verdünnungsmittels, wie z.B. Toluol, Tetrahydrofuran, Aceton, Methyl-isobutylketon oder Acetonitril, bei Temperaturen zwischen -80°C und 200°C, vorzugsweise zwischen -30°C und 120°C umsetzt.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana,

Leucophaea maderae, Blanella germanica, Acheta domesticus, Gryllotalpa spp.,

Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Chenocephalides spp., Pulex spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Verbindungen der Formel (I) zeigen auch eine fungizide Wirksamkeit.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Genannt seien die folgenden Verbindungen:

Acrinathrin, Alphamethrin, Betacyfluthrin, Bifenthrin, Brofenprox, Cis-Resmethrin, Clocythrin, Cycloprothrin, Cyfluthrin, Cyhalothrin, Cypermethrin, Deltamethrin, Esfenvalerate, Etofenprox, Fenpropathrin, Fenvalerate, Flucythrinate, Fluvalinate, Lambda-Cyhalothrin, Permethrin, Pyresmethrin, Pyrethrum, Silafluofen, Tralomethrin, Zetamethrin,
Alanycarb, Bendiocarb, Benfuracarb, Bufencarb, Butocarboxim, Carbaryl, Cartap, Ethiofencarb, Fenobucarb, Fenoxycarb, Isoprocarb, Methiocarb, Methomyl, Metolcarb, Oxamyl, Pirimicarb, Promecarb, Propoxur, Terbam, Thiodicarb, Thiofanox, Trimethacarb, XMC, Xylylcarb,
Acephate, Azinphos A, Azinphos M, Bromophos A, Cadusafos, Carbophenothion, Chlorfenvinphos, Chlormephos, Chlorpyrifos, Chlorpyrifos M, Cyanophos. Demeton M, Demeton-S-methyl, Demeton S, Diazinon, Dichlorvos, Dicliphos, Dichlorfenthion, Dicrotophos, Dimethoate, Dimethylvinphos, Dioxathion, Disulfoton, Edifenphos, Ethion, Etrimphos, Fenitrothion, Fenthion, Fonophos, Formothion, Heptenophos, Iprobenfos, Isazophos, Isoxathion, Phorate, Malathion, Mecarbam, Mervinphos, Mesulfenphos, Methacrifos, Methamidophos, Naled, Omethoate, Oxydemeton M, Oxydeprofos, Parathion A, Parathion M, Phenthoate, Phorate, Phosalone, Phosmet, Phosphamdon, Phoxim, Pirimiphos A, Pirimiphos M, Propaphos, Prothiophos, Prothoate, Pyraclophos, Pyridaphenthion, Quinalphos, Salithion, Sebufos, Sulfotep, Sulprofos, Tetrachlorvinphos, Temephos, Thiomethon, Thionazin, Trichlorfon, Triazophos, Vamidothion, Buprofezin, Chlorfluazuron, Diflubenzuron, Flucycloxuron, Flufenoxuron, Hexaflumuron, Pyriproxifen, Tebufenozide, Teflubenzuron, Triflumuron, Imidacloprid, Nitenpyram, N-[(6-Chloro-3-pyridinyl)methyl]-N'-cyano-N-methyl-ethanimidamid (NI-25), Abamectin, Amitrazin, Avermectin, Azadirachtin, Bensultap, Bacillus thuringiensis, Cyromazine, Diafenthiuron, Emamectin, Ethofenprox, Fenpyrad, Fipronil, Flufenprox, Lufenuron, Metaldehyd, Milbemectin, Pymetrozine, Tebufenpyrad, Triazuron, Aldicarb, Bendiocarb, Benfuracarb, Carbofuran, Carbosulfan, Chlorethoxyfos, Cloethocarb, Disulfoton, Ethophrophos, Etrimphos, Fenamiphos, Fipronil, Fonofos, Fosthiazate, Furathiocarb, HCH, Isazophos, Isofenphos, Methiocarb, Monocrotophos, Nitenpyram, Oxamyl, Phorate, Phoxim, Prothiofos, Pyrachlofos, Sebufos, Silafluofen, Tebupirimphos, Tefluthrin, Terbufos, Thiodicarb, Thiafenox, Azocyclotin, Butylpyridaben, Clofentezine, Cyhexatin, Diafenthiuron, Diethion, Emamectin, Fenazaquin, Fenbutatin Oxide, Fenothiocarb, Fenpropathrin, Fenpyrad, Fenpyroximate, Fluazinam, Fluazuron, Flucycloxuron, Flufenoxuron, Fluvalinate, Fubfenprox, Hexythiazox, Ivemectin, Methidathion, Monocrotophos, Moxidectin, Naled, Phosalone, Profenofos, Pyraclofos, Pyridaben, Pyrimidifen, Tebufenpyrad, Thuringiensin, Triarathene sowie 4-Bromo-2-(4-chlorophenyl)-1-(ethoxymethyl)-5-(trifluoromethyl)-1H-pyrrole-3-carbonitril (AC 303630).

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise. Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Die Herstellung und die Verwendung der erfindungegemäßen Stoffe wird durch die folgenden Beispiele veranschaulicht.

### Herstellungsbeispiele:

### Beispiel I-1

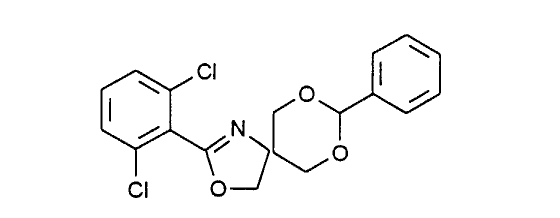

3,4 g (8,7 mmol) 2-Phenyl-5-(2,6-dichlorbenzoylamino)-5-hydroxymethyl-1,3-dioxan werden in 50 ml Toluol suspendiert und mit 2 g Thionylchlorid 24 Stunden bei 80°C gerührt. Dann werden überschüssiges Thionylchlorid und Toluol im Wasserstrahlvakuum abdestilliert.

Der Rückstand wird in 50 ml Toluol aufgenommen und mit 0,9 g Kalium-t-butylat versetzt. Das Reaktionsgemisch wird dann unter dünnschicht-chromatographischer Kontrolle bei 50°C gerührt. Nach vollständiger Umsetzung wird die erhaltene Lösung mit Wasser gewaschen und die organische Phase anschließend im Wasserstrahlvakuum eingeengt. Der Rückstand wird an Kieselgel mit Cyclohexan/Essigsäureethylester (Vol. 2:1) chromatographiert.

Man erhält 1 g (31% der Theorie) 8-Phenyl-2-(2,6-dichlor-phenyl)-1-aza-3,7,9-trioxaspiro[4.5]dec-1-en vom Schmelzpunkt 158°C - 160°C.

Gemäß den Verfahren a - d wurden die in der Tabelle 8 aufgeführten Verbindungen der Formel (I) hergestellt:

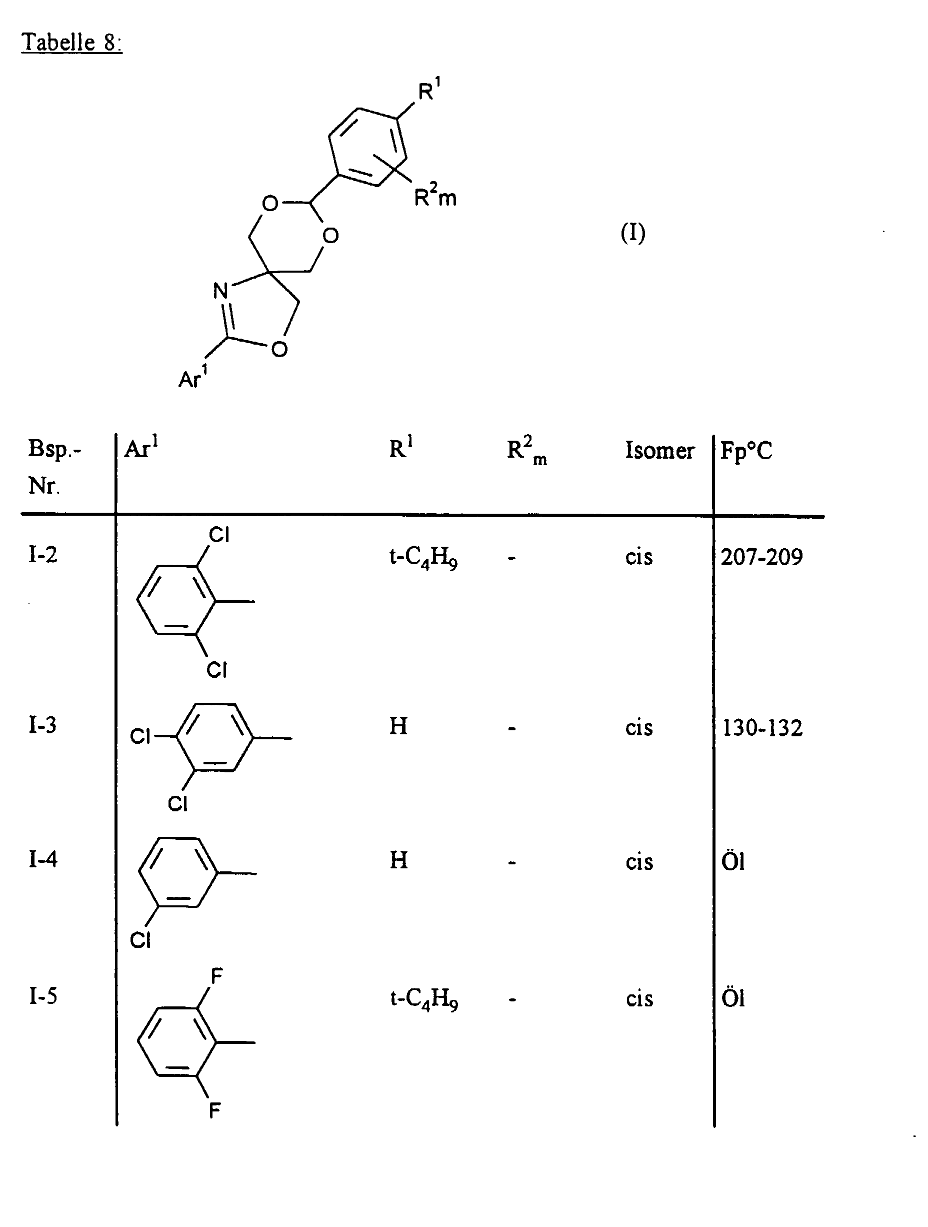

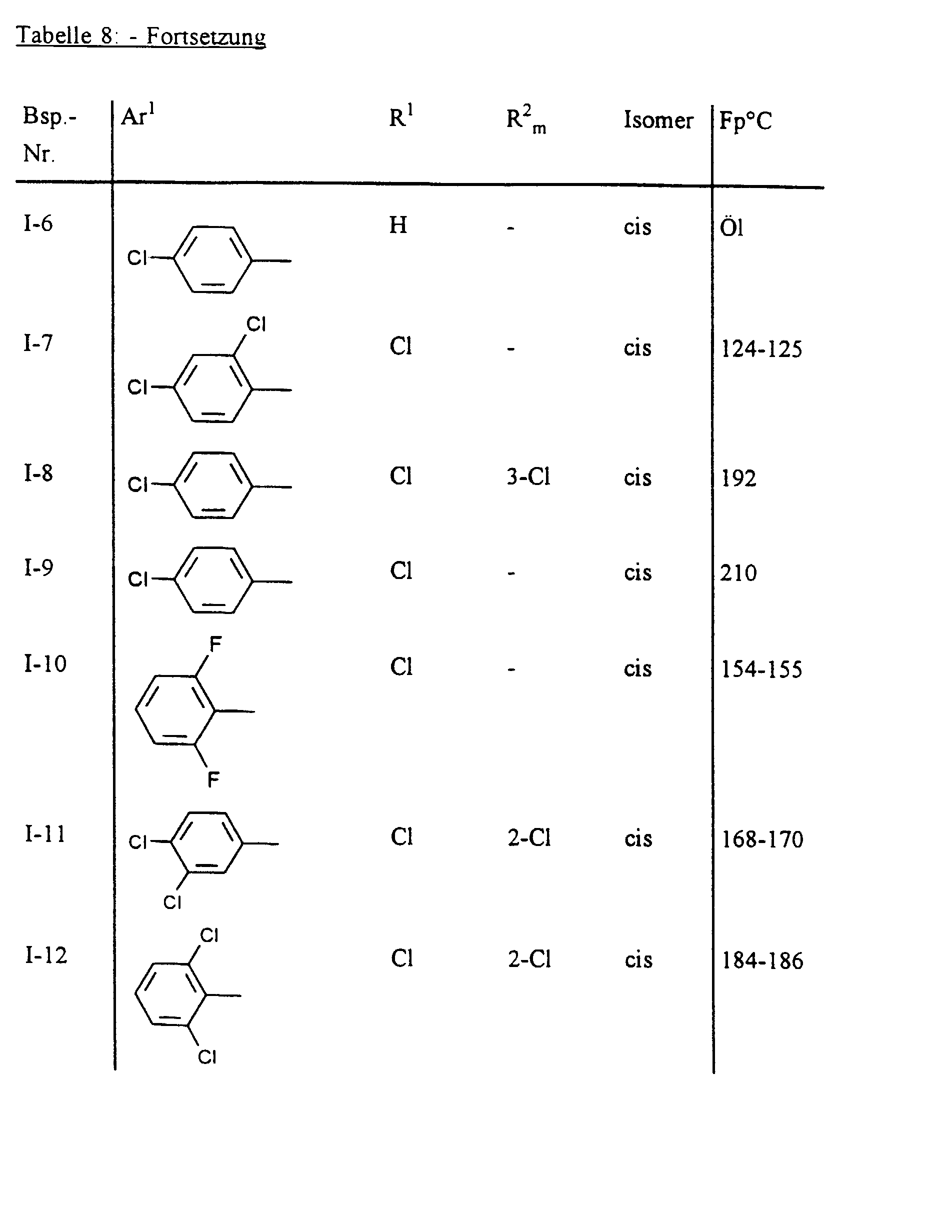

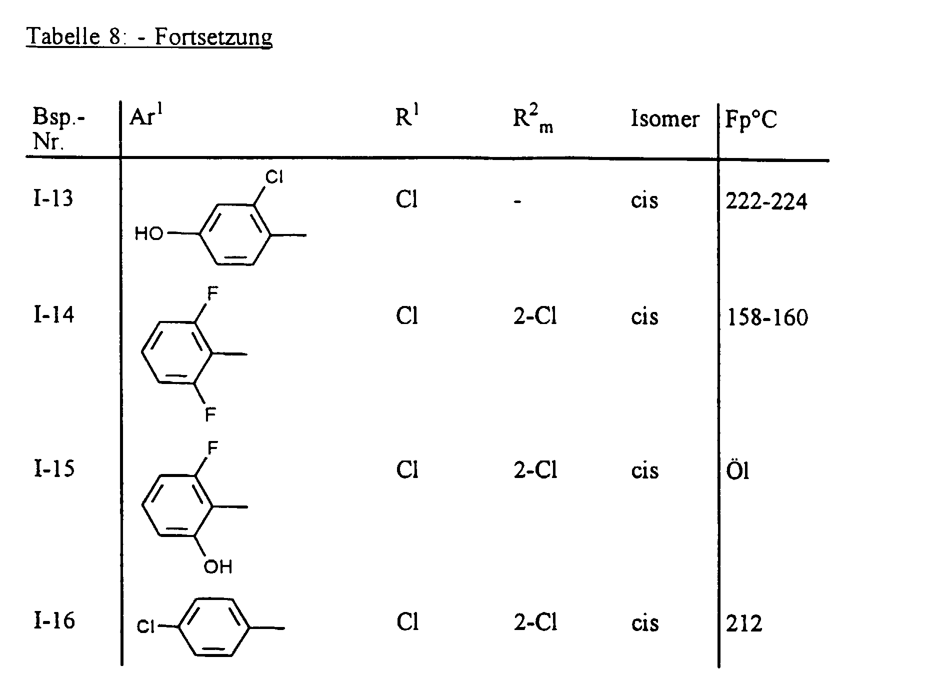

### Ausgangsstoffe der Formel (II):

### Beispiel (II-1)

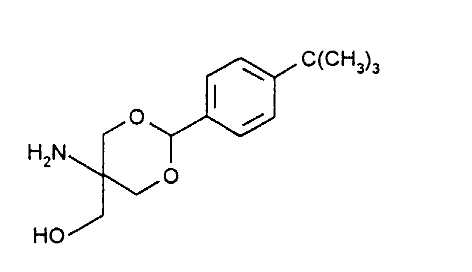

104 g (0,35 mol) 2-(4-t-Butyl-phenyl)-5-hydroxymethyl-5-nitro-1,3-dioxan werden in 1 Liter Ethanol mit 10 ml Triethylamin und 10 g Palladium/Kohle versetzt und 3 Tage bei 20°C und 30 bar hydriert. Dann wird filtriert, das Filtrat im Wasserstrahlvakuum eingeengt, der Rückstand mit n-Hexan digeriert und das hierbei kristallin erhaltene Produkt durch Absaugen isoliert.

Man erhält 43 g (47% der Theorie) (cis)-2-(4-t-Butyl-phenyl)-5-hydroxymethyl-5-amino-1,3-dioxan vom Schmelzpunkt 138°C - 140°C.

In Analogie zu Beispiel (II-1) wurden die in Tabelle 9 aufgeführten Verbindungen der Formel (II) erhalten:

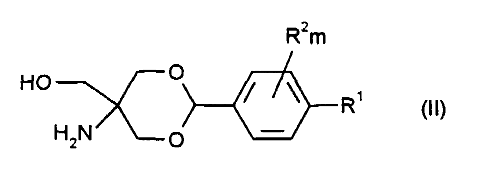

**Tabelle 9:**

| Bsp.-Nr | R¹ | R²ₘ | Isomer | Fp. °C |
|---|---|---|---|---|
| II-2 | Cl | - | trans | 149-150 |
| II-3 | Cl | - | cis | 168-170 |
| II-4 | t-C₄H₉ | - | trans | 176-178 |
| II-5 | Cl | 2-Cl | cis | 155 |

### Ausgangsstoffe der Formel (IV):

### Beispiel (IV-1)

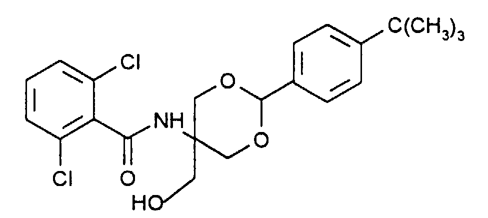

5 g (20 mmol) 2-(4-t-Butyl-phenyl)-5-amino-5-hydroxymethyl-1,3-dioxan werden in 80 ml Methylenchlorid mit 5 ml Triethylamin versetzt. Dann wird bei 0°C eine Lösung von 4,5 g (20 mmol) 2,6-Dichlor-benzoylchlorid in 5 ml Methylenchlorid zugetropft und die Mischung wird 15 Stunden bei 20°C gerührt. Anschließend wird mit 0,5-N Natronlauge gewaschen, die organische Phase mit Natriumsulfat getrocknet und filtriert. Das Filtrat wird im Wasserstrahlvakuum eingeengt, der Rückstand durch Digerieren mit n-Hexan zur Kristallisation gebracht und das Produkt durch Absaugen isoliert.

Man erhält 7,2 g (83% der Theorie) 2-(4-t-Butyl-phenyl)-5-hydroxymethyl-5-(2,6-dichlor-benzoyl-amino)-1,3-dioxan vom Schmelzpunkt 178°C - 180°C.

Analog zu Beispiel (IV-1) ließen sich auch die in der nachstehenden Tabelle 10 aufgeführten Verbindungen der Formel (IV) herstellen.

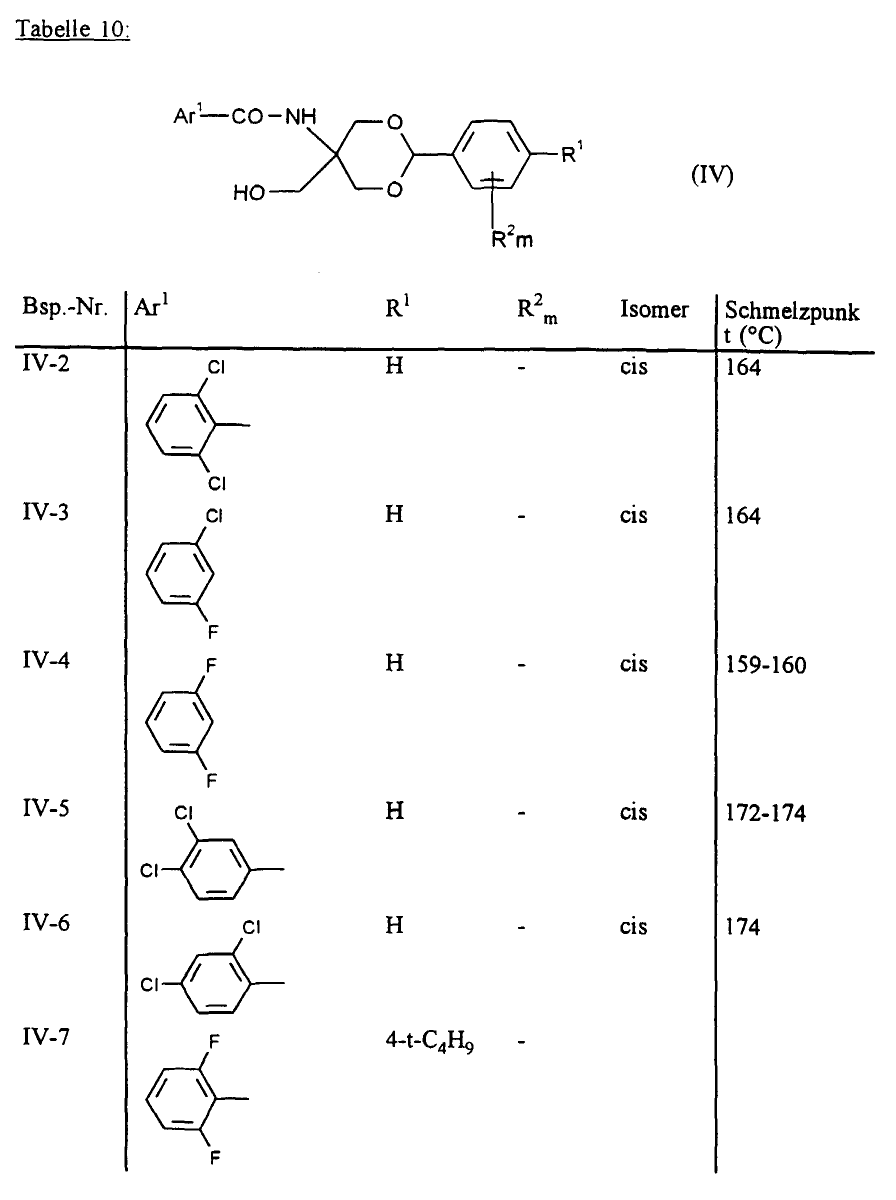

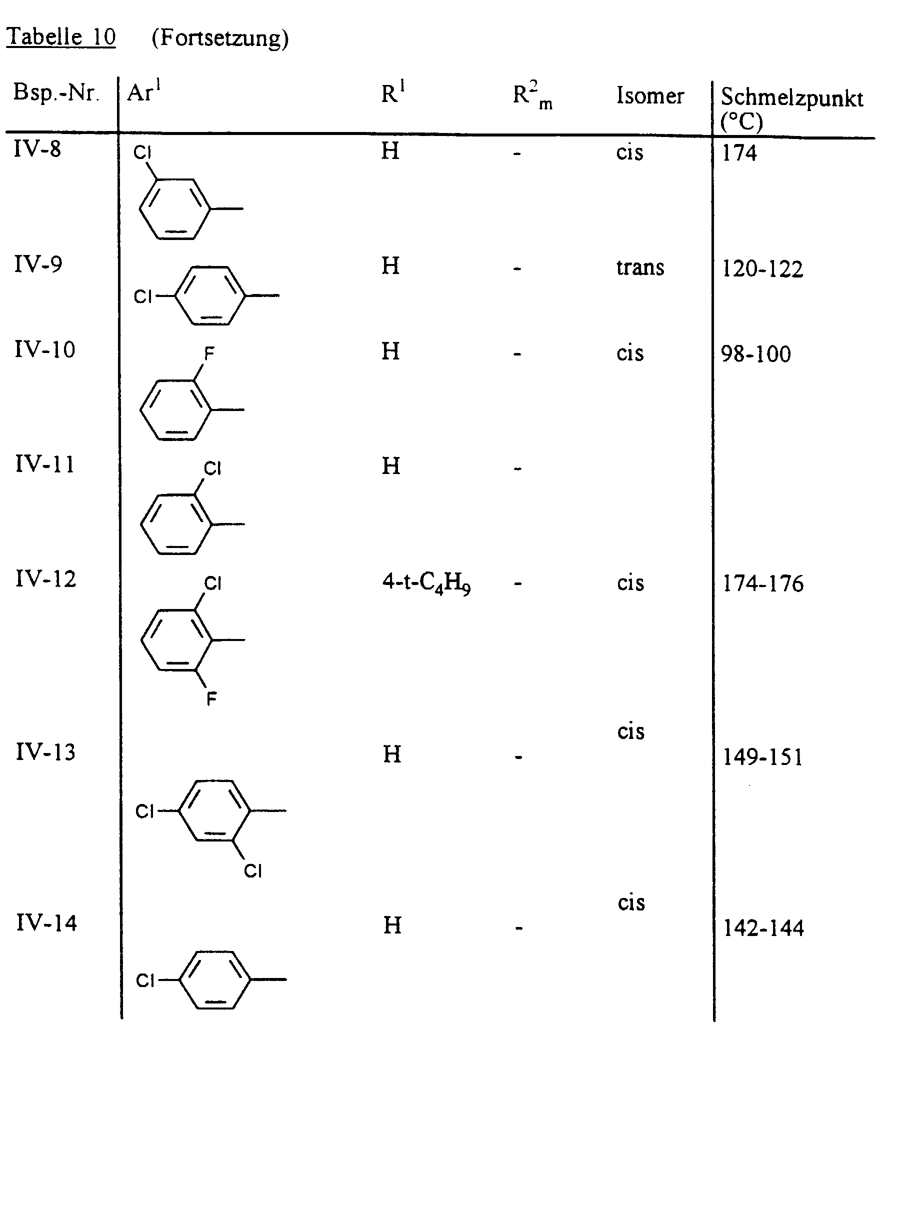

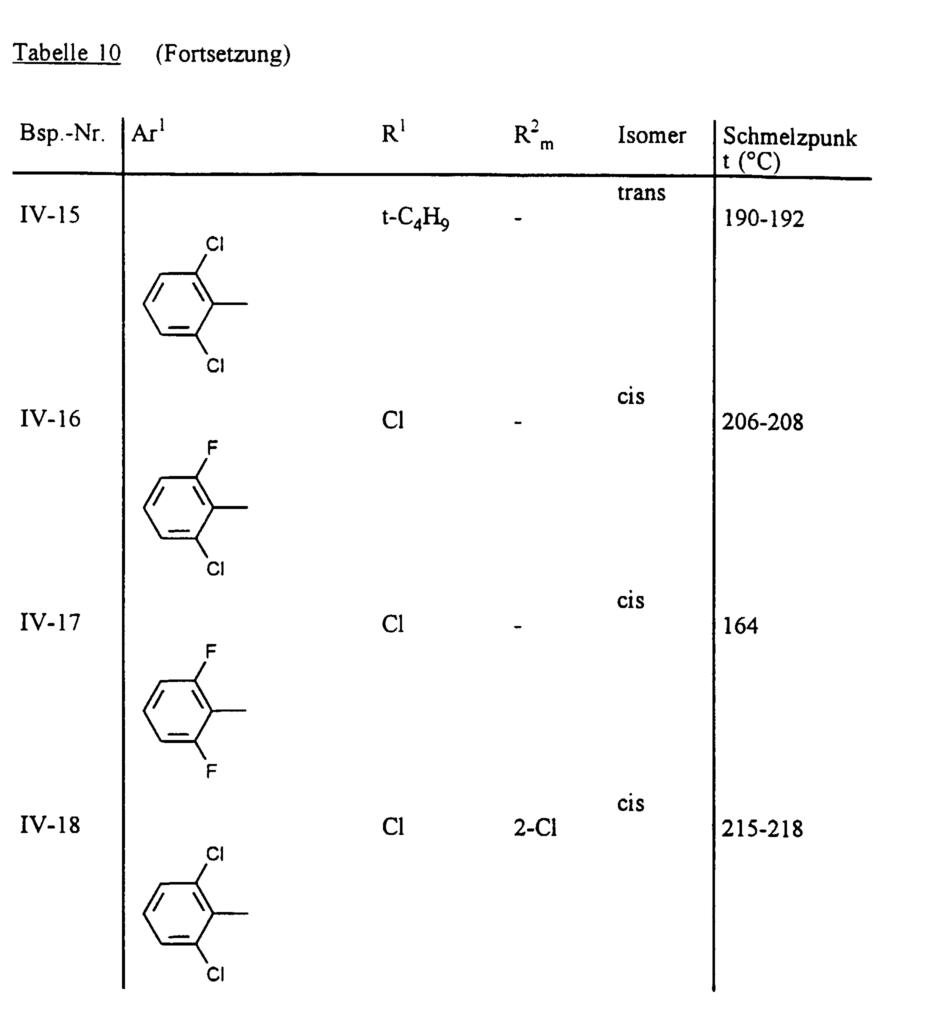

### Beispiel VI-1:

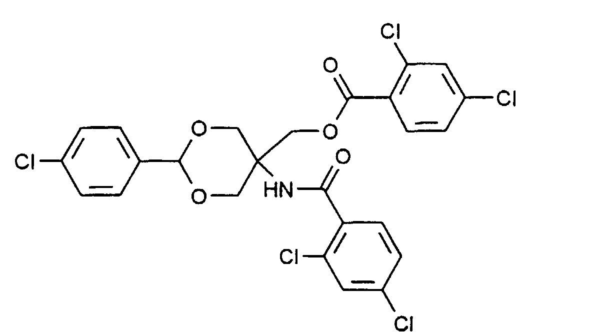

4,1 g (15 mmol) 2-(4-Chlorphenyl)-5-amino-5-hydroxymethyl-1,3-dioxan werden in 100 ml Methylenchlorid mit 5 ml Triethylamin versetzt und bei 0°C 6,3 g (30 mmol) 2,4-Dichlorbenzoylchlorid in 5 ml Methylenchlorid zugetropft. Man rührt 15 Stunden bei Raumtemperatur, wäscht das Reaktionsgemisch mit Wasser, trocknet die organische Phase mit Magnesiumsulfat, engt im Vakuum ein und fällt den Rückstand durch Digerieren mit n-Hexan aus.
Man erhält 8,3 g ( ca. 94 % der Theorie) der oben gezeigten Verbindung vom Schmelzpunkt 148-150°C.

Analog zu Beispiel (VI-1) erhält man die in der Tabelle 11 aufgeführten Verbindungen:

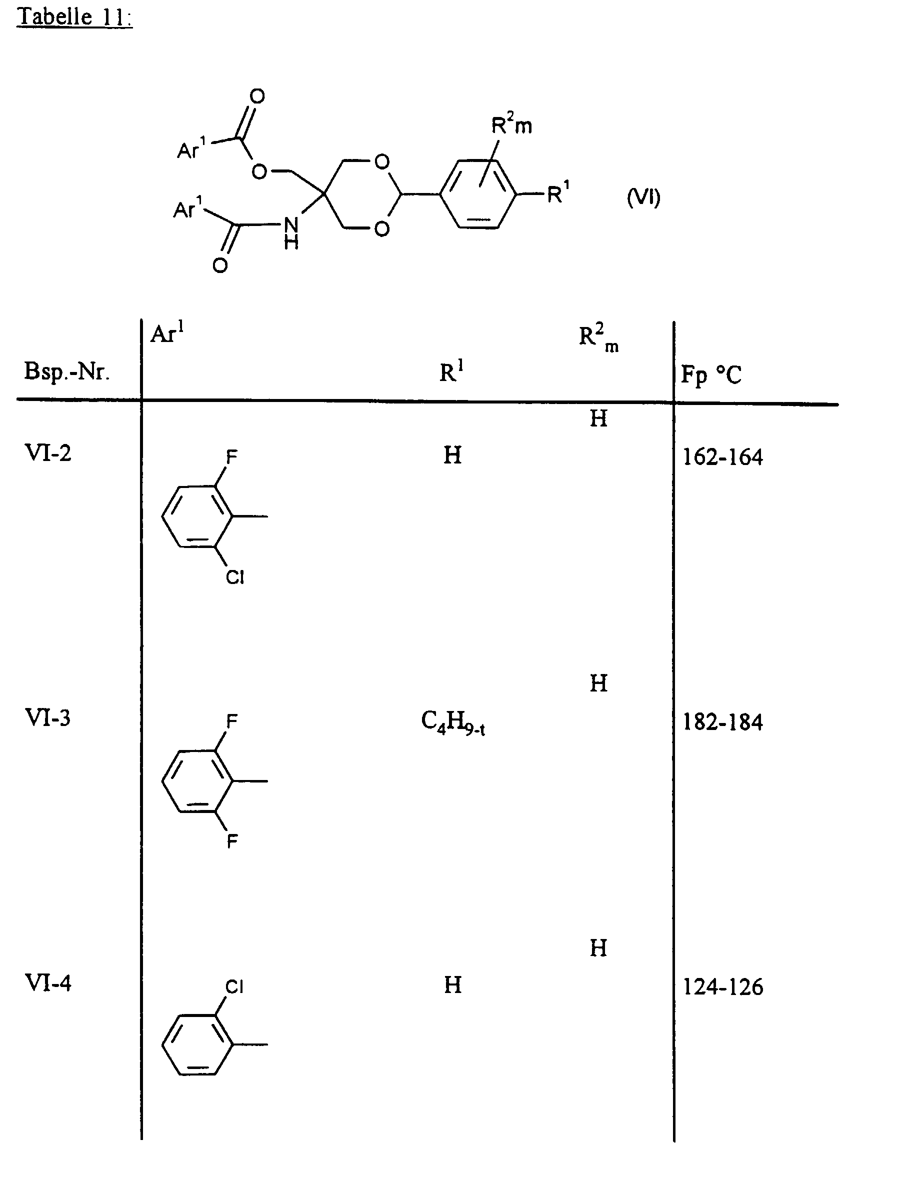

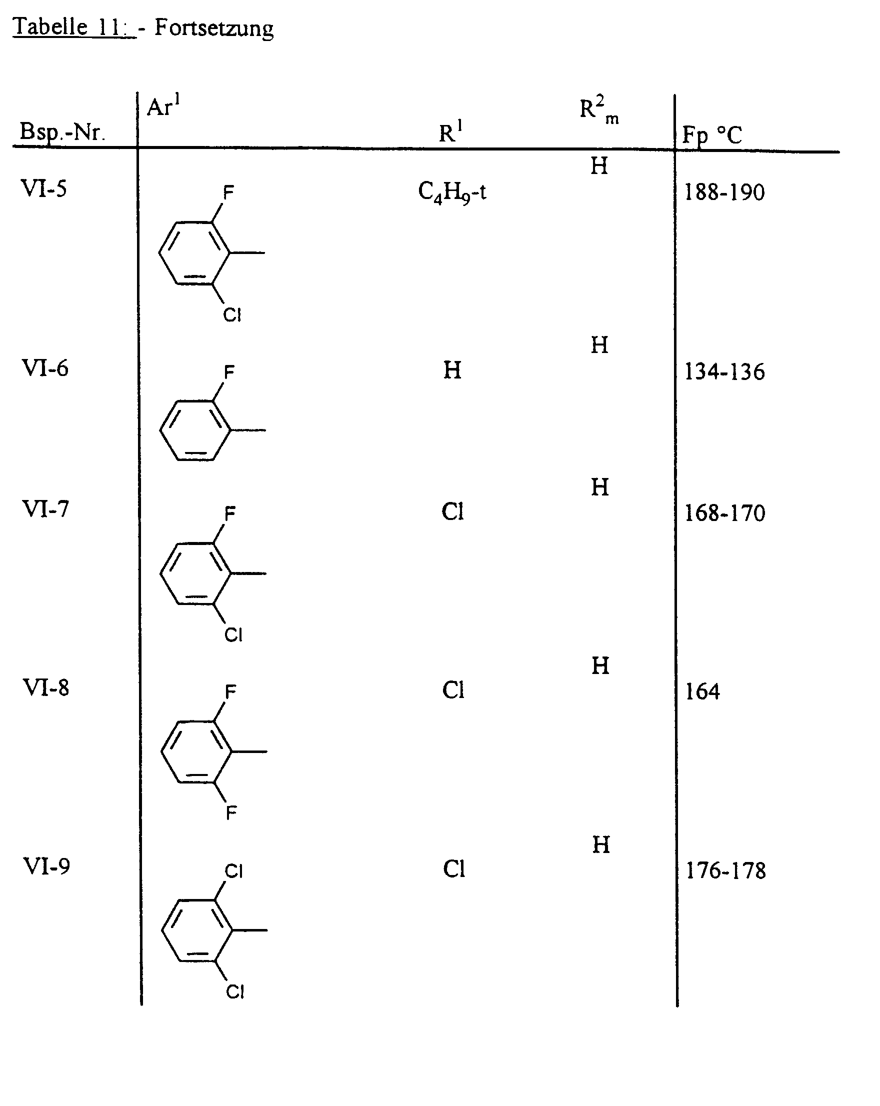

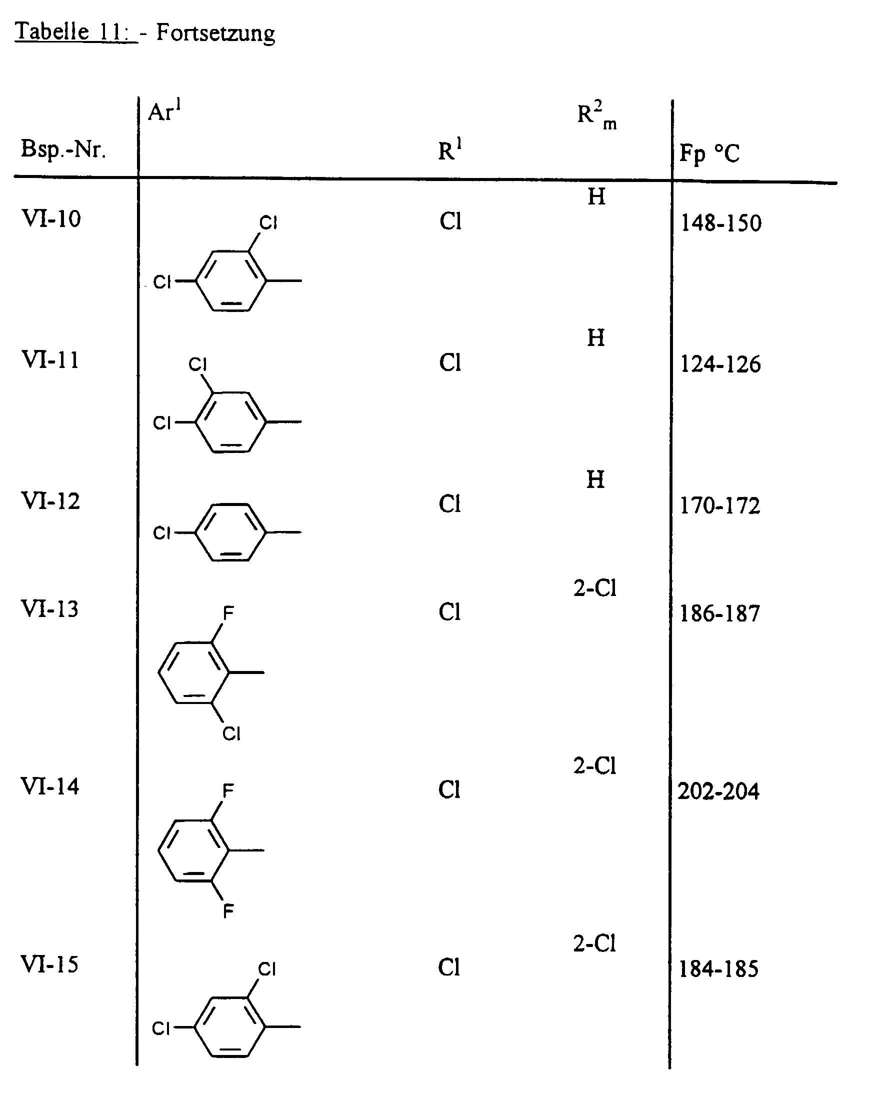

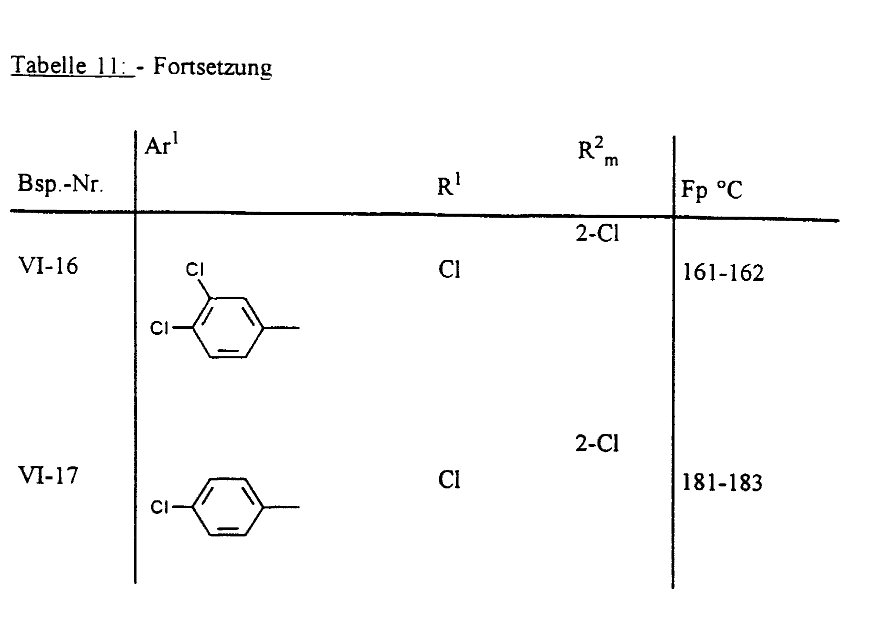

### Ausgangsstoffe der Formel (VII):

### Beispiel (VII-1)

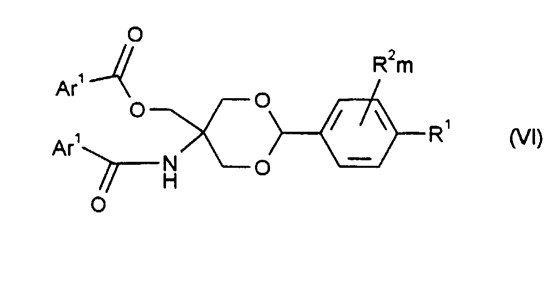

81 g (0,5 mol) 4-t-Butyl-benzaldehyd werden in 500 ml Toluol vorgelegt, mit 5 g p-Toluol-sulfonsäure versetzt und nach Zugabe von 76 g (0,5 mol) Tris-hydroxymethylnitromethan am Wasserabscheider so lange erhitzt, bis die Wasserabscheidung beendet ist. Das Reaktionsgemisch wird dann mit 5%iger Natriumhydrogencarbonat-Lösung gewaschen und anschließend eingeengt. Der Rückstand wird aus Toluol/Cyclohexan umkristallisiert.

Man erhält 116 g (78% der Theorie) 2-(4-t-Butyl-phenyl)-5-hydroxymethyl-5-nitro-1,3 dioxan vom Schmelzpunkt 90°C - 92°C.

In Analogie zu Beispiel (VII-1) wurden die in Tabelle 12 aufgeführten Verbindungen der Formel (VII) erhalten:

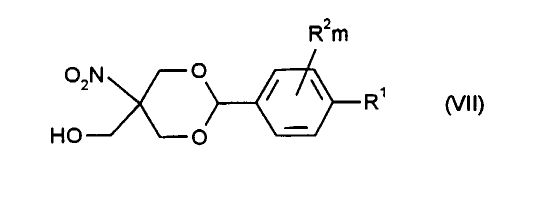

**Tabelle 12**

| Bsp.-Nr. | R¹ | R²ₘ | Isomer | Fp. °C |
|---|---|---|---|---|
| VII-2 | C₄H₉-t | - | trans | 143-145 |
| VII-3 | Cl | 2-Cl | cis | 141-142 |

### Anwendungsbeispiele:

### Beispiel A

### Tetranychus-Test (OP-resistent/Tauchbehandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100%, daß alle Spinnmilben abgetötet wurden; 0% bedeutet, daß keine Spinnmilben abgetötet wurden.

Bei diesem Test zeigen z.B. die Verbindungen der Herstellungsbeispiele I-9 und I-11 bei einer Wirkstoffkonzentration von 0,01% und I-16 bei 0,02% nach 7 Tagen einen Abtötungsgrad von 98%.

### Beispiel B

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichbtattkäfer-Larven Phaedon cochleariae besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigt z.B. die Verbindung von Herstellungsbeispiel I-10 bei einer beispielhaften Wirkstoffkonzentration von 0,1% einen Abtötungsgrad von 100 % nach 7 Tagen.

### Beispiel C

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

In diesem Test bewirkte z.B. die Verbindung gemäß Herstellungsbeispiel I-2 bei einer beispielhaften Wirkstoffkonzentration von 0,1% eine Abtötung von 100 % nach 7 Tagen.

### Beispiel D

### Spodoptera-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

In diesem Test Bewirkte z.B. die Verbindung gemäß Herstellungsbeispiel I-11 bei einer beispielhaften Wirkstoffkonzentration von 0,1% einen Abtötungsgrad von 100 % nach 7 Tagen.

## Patentansprüche

1. Azatrioxaspiroalkene der Formel (I), 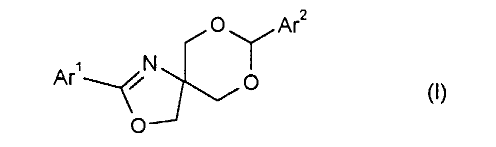 in welcher
Ar¹ für Phenyl steht, das gegebenenfalls einfach bis fünffach, gleich oder verschieden substituiert ist durch Halogen, Cyano, Nitro, Hydroxy, C₁-C₆-Alkyl, C₁-C₆-Alkoxy, C₁-C₆-Alkylthio, C₁-C₆-Halogenalkyl, C₁-C₆-Halogenalkoxy oder C₁-C₆-Halogenalkylthio,
Ar² für Phenyl steht, das gegebenenfalls einfach bis fünffach, gleich oder verschieden substituiert ist durch Halogen, Cyano, Nitro, Hydroxy, C₁-C₁₈-Alkyl, C₁-C₈-Halogenalkyl, C₁-C₈-Alkoxy-C₁-C₈-Alkyl, C₁-C₁₈-Alkoxy (bei dem gegebenenfalls einzelne Methylengruppen durch Sauerstoffatome ersetzt sind), C₁-C₈-Halogenalkoxy, C₁-C₁₈-Alkylthio, C₁-C₈-Halogenalkylthio, C₁-C₈-Alkyl-carbonyl, C₁-C₈-Halogenalkylcarbonyl, C₁-C₈-Alkoxy-carbonyl, C₁-C₈-Halogenalkoxy-carbonyl, C₁- C₃-Alkylendioxy, C₁-C₃-Halogenalkylendioxy, jeweils gegebenenfalls durch C₁-C₆-Alkyl, C₁-C₆-Alkoxy, Cyclohexyl oder Phenyl substituiertes Cyclohexyl, Cyclohexylmethyl oder Cyclohexyloxy, gegebenenfalls einfach oder zweifach, gleich oder verschieden durch Halogen, C₁-C₄-Alkyl oder C₁-C₄-Halogenalkyl substituiertes Pyridyloxy, jeweils gegebenenfalls einfach bis vierfach, gleich oder verschieden durch Halogen, C₁-C₆-Alkyl, C₁-C₆-Halogenalkyl, C₁-C₆-Alkoxy, C₁-C₆-Hal ogenalkoxy,C₁-C₆-Alkoxy-C₁-C₆-alkyl, C₁-C₆-Alkoxy-ethylenoxy, C₁-C₆-Alkylthio oder C₁-C₆-Halogenalkylthio substituiertes Phenyl, Benzyl, Benzoyl, Phenoxy, Phenylthio, Benzyloxy oder Benzylthio,
deren Isomerengemische sowie deren reinen E- oder Z-Isomeren.

2. Azatrioxaspiroalkene der allgemeinen Formel (I) gemäß Anspruch 1, in welcher
Ar¹ für Phenyl steht, das gegebenenfalls einfach bis dreifach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio,
Ar² für Phenyl steht, das gegebenenfalls einfach bis vierfach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, n-, i-, s- oder t-Butyl, n-, i-, s-oder t-Pentyl, n-, i-, s- oder t-Hexyl, n oder i-Octyl, n- oder i-Nonyl, n- oder i-Decyl, n- oder i-Dodecyl, Methoxy, Ethoxy, n- oder i-Propoxy, n-, i-, s- oder t-Butoxy, n-, i-, s- oder t-Pentyloxy, n-, i-, s- oder t-Hexyloxy, n- oder i-Octyloxy, n- oder i-Nonyloxy, n- oder i-Decyloxy, n- oder i-Dodecyloxy, Methylthio, Ethylthio, n- oder i-Propylthio, n-, i-, s- oder t-Butylthio, n-, i-, s- oder t-Pentylthio, n-, i-, s-oder t-Hexylthio, n- oder i-Ocytylthio, n- oder i-Nonylthio, n- oder i-Decylthio, n- oder i-Dodecylthio, Acetyl, Propionyl, n- oder i-Butyroyl, n-, i-, s- oder t-Valeroyl, Methoxycarbonyl, Ethoxycarbonyl, n- oder i-Propoxycarbonyl, n-, i-, s- oder t-Butoxycarbonyl, n-, i-, s-oder t-Pentoxycarbonyl, Methylendioxy, Ethylendioxy, jeweils gegebenenfalls durch Methyl, Ethyl, n- oder i-Propyl, n-, i-, s- oder t-Butyl, Methoxy, Ethoxy, n- oder i-Propoxy, n-, i-, s- oder t-Butoxy, Cyclohexyl oder Phenyl substituiertes Cyclohexyl, Cyclohexylmethyl oder Cyclohexyloxy, gegebenenfalls einfach bis dreifach, gleich oder verschieden durch Fluor, Chlor, Brom, Methyl, Ethyl, n- oder i-Propyl, n-, i-, s- oder t-Butyl, oder durch Trifluormethyl substituiertes Pyridyloxy, jeweils gegebenenfalls einfach bis dreifach, gleich oder verschieden durch Fluor, Chlor, Brom, Methyl, Ethyl, n- oder i-Propyl, n-, i-, s- oder t-Butyl, Trifluormethyl, Methoxy, Ethoxy, n-oder i-Propoxy, n-, i-, s- oder t-Butoxy, Difluormethoxy, Trifluormethoxy, Methyl-thio, Ethylthio, n- oder i-Propylthio, n-, i-, s- oder t-Butylthio, Difluormethylthio oder Trifluormethylthio substituiertes Phenyl, Benzyl, Benzoyl, Phenoxy, Phenylthio, Benzyloxy oder Benzylthio.

3. Azatrioxyspiroalkene der Formel (I) gemäß Anspruch 1, in welcher
Ar¹ für Phenyl steht, das gegebenenfalls einfach bis zweifach, gleich oder verschieden durch Fluor, Chlor, Brom oder Hydroxy, Methyl, Ethyl, n- oder i-Propyl, Methoxy, Ethoxy, n- oder i-Propoxy, Trifluormethyl oder Trifluormethoxy substituiert ist und
Ar² für Phenyl steht, das gegebenenfalls einfach bis dreifach, gleich oder verschieden substituiert ist durch Fluor, Chlor, Brom, Cyano, Nitro, Hydroxy, jeweils gegebenenfalls durch Fluor und/oder Chlor substituiertes Methyl, Ethyl, n- oder i-Propyl, n-, i-, s- oder t-Butyl, n-, i-, s-oder t-Pentyl, n-, i-, s- oder t-Hexyl, n oder i-Octyl, n- oder i-Nonyl, n- oder i-Decyl, n- oder i-Dodecyl, Methoxy, Ethoxy, n- oder i-Propoxy, n-, i-, s- oder t-Butoxy, n-, i-, s- oder t-Pentyloxy, n-, i-, s- oder t-Hexyloxy, n- oder i-Octyloxy, n- oder i-Nonyloxy, n- oder i-Decyloxy, n- oder i-Dodecyloxy, Methylthio, Ethylthio, n- oder i-Propylthio, n-, i-, s- oder t-Butylthio, n-, i-, s- oder t-Pentylthio, n-, i-, s-oder t-Hexylthio, n- oder i-Ocytylthio, n- oder i-Nonylthio, n- oder i-Decylthio, n- oder i-Dodecylthio, Acetyl, Propionyl, n- oder i-Butyroyl, n-, i-, s- oder t-Valeroyl, Methoxycarbonyl, Ethoxycarbonyl, n- oder i-Propoxycarbonyl, n-, i-, s- oder t-Butoxycarbonyl, n-, i-, s-oder t-Pentoxycarbonyl, Methylendioxy, Ethylendioxy, jeweils gegebenenfalls durch Methyl, Ethyl, n- oder i-Propyl, n-, i-, s- oder t-Butyl, Methoxy, Ethoxy, n- oder i-Propoxy, n-, i-, s- oder t-Butoxy, Cyclohexyl oder Phenyl substituiertes Cyclohexyl, Cyclohexylmethyl oder Cyclohexyloxy, gegebenenfalls einfach bis dreifach, gleich oder verschieden durch Fluor, Chlor, Brom, Methyl, Ethyl, n- oder i-Propyl, n-, i-, s- oder t-Butyl, oder durch Trifluormethyl substituiertes Pyridyloxy, jeweils gegebenenfalls einfach bis dreifach, gleich oder verschieden durch Fluor, Chlor, Brom, Methyl, Ethyl, n- oder i-Propyl, n-, i-, s- oder t-Butyl, Trifluormethyl, Methoxy, Ethoxy, n-oder i-Propoxy, n-, i-, s- oder t-Butoxy, Difluormethoxy, Trifluormethoxy, Methyl-thio, Ethylthio, n- oder i-Propylthio, n-, i-, s- oder t-Butylthio, Difluormethylthio oder Trifluormethylthio substituiertes Phenyl, Benzyl, Benzoyl, Phenoxy, Phenylthio, Benzyloxy oder Benzylthio.

4. Verfahren zur Herstellung von Azatrioxaspiroalkenen der Formel (I), gemäß Anspruch 1, dadurch gekennzeichnet, daß man
(a) Aminoalkohole der Formel (II) 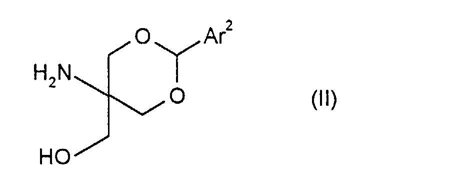 in welcher
Ar² die im Anspruch 1 angegebene Bedeutung hat,
mit Carbonsäuren der allgemeinen Formel (III)
Ar¹-COOH (III)
in welcher
Ar¹ die im Anspruch 1 angegebene Bedeutung hat,
mit einem wasserentziehenden Mittel gegebenenfalls in Gegenwart eines Verdünnungsmittels umsetzt, oder daß man
(b) Amidalkohole der Formel (IV) 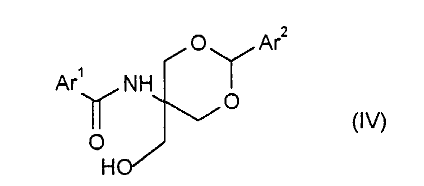 in welcher
Ar¹ und Ar² die oben angegebene Bedeutung haben,
mit einem wasserentziehenden Mittel gegebenenfalls in Gegenwart eines Verdünnungsmittels umsetzt, oder daß man
(c) Carbonsäureamide der Formel (V) 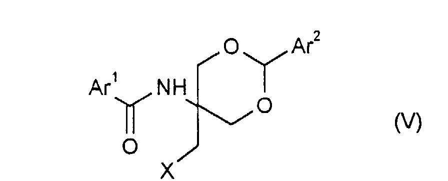 in welcher
Ar¹ und Ar² die oben angegebene Bedeutung haben und
X für Halogen, Alkylsulfonyloxy oder Arylsulfonyloxy steht,
mit einer Base gegebenenfalls in Gegenwart eines Verdünnungsmittels umsetzt., oder daß man
d) Carboxamidbenzoate der Formel (VI) 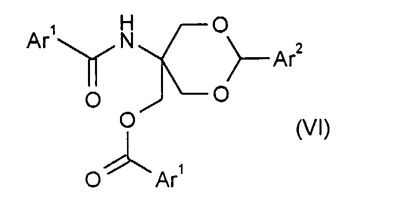 in welcher
Ar¹ und Ar² die oben angegebene Bedeutung haben,
mit einer Base gegebenenfalls in Gegenwart eines Verdünnungsmittels umsetzt.

5. Schädlingsbekämpfungsmittel, gekennzeichnet durch einen Gehalt an mindestens einer Verbindung der Formel (I) gemäß Anspruch 1.

6. Verfahren zur Bekämpfung von tierischen Schädlingen, dadurch gekennzeichnet, daß man Verbindungen der Formel (I) gemäß Anspruch 1 auf tierische Schädlinge und/oder deren Lebensraum einwirken läßt.

7. Verwendung von Verbindungen der Formel (I) gemäß Anspruch 1 zur Bekämpfung von tierischen Schädlingen.

8. Verfahren zur Herstellung von Schädlingsbekämpfungsmitteln, dadurch gekennzeichnet, daß man Verbindungen der Formel (I) gemäß Anspruch 1 mit Streckmitteln und/oder oberflächenaktiven Mitteln vermischt.

9. Aminoalkohole der Formel (II) 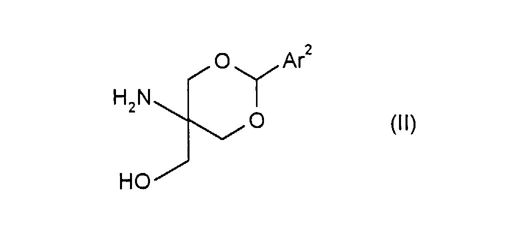 in welcher
Ar² die oben angegebene Bedeutung hat, mit Ausnahme der Verbindung der Formel (II), bei der Ar² für Phenyl steht.

10. Amidalkohole der Formel (IV) 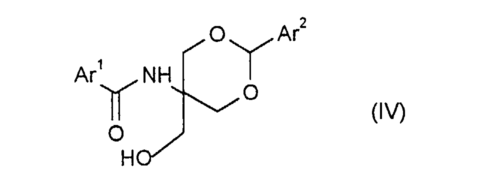 in welcher
Ar¹ und Ar² gleich oder verschieden sind und die oben angegebenen Bedeutungen haben.

11. Carbonsäureamide der Formel (V) 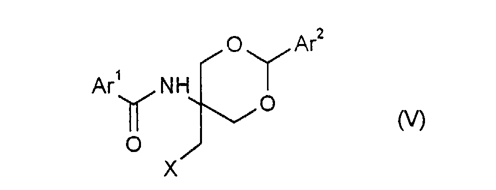 in welcher
Ar¹ und Ar² gleich oder verschieden sind und die oben angegebenen Bedeutungen haben
und
X für Halogen, Alkylsulfonyloxy oder Arylsulfonyloxy steht.

12. Nitroalkohole der Formel (VII) 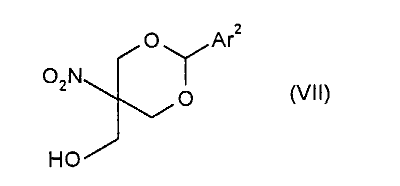 in welcher
Ar² die oben angegebene Bedeutung hat mit Ausnahme der Verbindungen der Formel (VI), in welcher Ar² für Phenyl, 4-Methoxy-phenyl, 4-Cyano-phenyl, 2-Nitro-phenyl, 3-Nitro-phenyl, 4-Nitro-phenyl, 4-Chlor-phenyl, 4-Dimethylamino-phenyl, 4-Hydroxy-phenyl, 2-Hydroxy-phenyl, 3-Ethoxy-4-hydroxy-phenyl oder 4-Hydroxy-3-methoxy-phenyl.

13. Carboxamidbenzoate der Formel (VI) 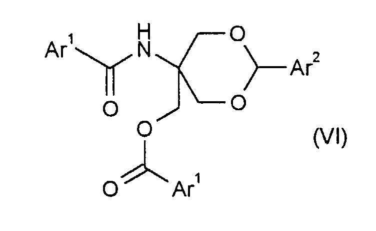 in welcher
Ar¹ und Ar² die oben angegebene Bedeutung haben.

## Claims

1. Azatrioxaspiroalkene of the formula (I) 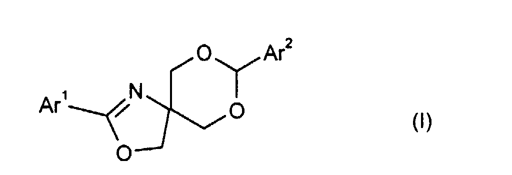 in which
Ar¹ represents phenyl which is optionally monosubstituted to pentasubstituted by identical or different substituents from the series consisting of halogen, cyano, nitro, hydroxyl, C₁-C₆-alkyl, C₁-C₆-alkoxy, C₁-C₆-alkylthio, C₁-C₆-halogenoalkyl, C₁-C₆-halogenoalkoxy or C₁-C₆-halogenoalkylthio,
Ar² represents phenyl which is optionally monosubstituted to pentasubstituted by identical or different substituents from the series consisting of halogen, cyano, nitro, hydroxyl, C₁-C₁₈-alkyl, C₁-C₈-halogenoalkyl, C₁-C₈-alkoxy-C₁-C₈-alkyl, C₁-C₁₈-alkoxy (in which individual methylene groups are optionally replaced by oxygen atoms) , C₁-C₈-halogenoalkoxy, C₁-C₁₈-alkylthio, C₁-C₈-halogenoalkylthio, C₁-C₈-alkyl-carbonyl, C₁-C₈-halogenoalkylcarbonyl, C₁-C₈-alkoxy-carbonyl, C₁-C₉-halogenoalkoxy-carbonyl, C₁-C₃-alkylenedioxy, C₁-C₃-halogenoalkylenedioxy, or cyclohexyl, cyclohexylmethyl or cyclohexyloxy, each of which is optionally substituted by C₁-C₆-alkyl, C₁-C₆-alkoxy, cyclohexyl or phenyl, or pyridyloxy which is optionally monosubstituted or disubstituted by identical or different substituents from the series consisting of halogen, C₁-C₄-alkyl or C₁-C₄-halogenoalkyl, or phenyl, benzyl, benzoyl, phenoxy, phenylthio, benzyloxy or benzylthio, each of which is optionally monosubstituted to tetrasubstituted by identical or different substituents from the series consisting of halogen, C₁-C₆-alkyl, C₁-C₆-halogenoalkyl, C₁-C₆-alkoxy, C₁-C₆-halogenoalkoxy, C₁-C₆-alkoxy-C₁-C₆-alkyl, C₁-C₆-alkoxy-ethyleneoxy, C₁-C₆-alkylthio or C₁-C₆-halogenoalkylthio,
its isomer mixtures and its pure E or Z isomers.

2. Azatrioxaspiroalkene of the general formula (I) according to Claim 1, in which
Ar¹ represents phenyl which is optionally monosubstituted to trisubstituted by identical or different substituents from the series consisting of fluorine, chlorine, bromine, cyano, nitro, hydroxyl, or methyl, ethyl, n- or i-propyl, methoxy, ethoxy, n- or i-propoxy, methylthio, ethylthio, n- or i-propylthio, each of which is optionally substituted by fluorine and/or chlorine,
Ar² represents phenyl which is optionally monosubstituted to tetrasubstituted by identical or different substituents from the series consisting of fluorine, chlorine, bromine, cyano, nitro, hydroxyl, or methyl, ethyl, n- or i-propyl, n-, i-, s- or t-butyl, n-, i-, s- or t-pentyl, n-, i-, s- or t-hexyl, n- or i-octyl, n- or i-nonyl, n- or i-decyl, n- or i-dodecyl, methoxy, ethoxy, n- or i-propoxy, n-, i-, s- or t-butoxy, n-, i-, s- or t-pentyloxy, n-, i-, s-or t-hexyloxy, n- or i-octyloxy, n- or i-nonyloxy, n- or i-decyloxy, n- or i-dodecyloxy, methylthio, ethylthio, n- or i-propylthio, n-, i-, s- or t-butylthio, n-, i-, s- or t-pentylthio, n-, i-, s- or t-hexylthio, n- or i-octylthio, n- or i-nonylthio, n- or i-decylthio, nor i-dodecylthio, acetyl, propionyl, n- or i-butyroyl, n-, i-, s- or t-valeroyl, methoxycarbonyl, ethoxycarbonyl, n- or i-propoxycarbonyl, n-, i-, s- or t-butoxycarbonyl, n-, i-, s- or t-pentoxycarbonyl, methylenedioxy, ethylenedioxy, each of which is optionally substituted by fluorine and/or chlorine, or cyclohexyl, cyclohexylmethyl or cyclohexyloxy, each of which is optionally substituted by methyl, ethyl, n- or i-propyl, n-, i-, s- or t-butyl, methoxy, ethoxy, n- or i-propoxy, n-, i-, s- or t-butoxy, cyclohexyl or phenyl, or pyridyloxy which is optionally monosubstituted to trisubstituted by identical or different substituents from the series consisting of fluorine, chlorine, bromine, methyl, ethyl, nor i-propyl, n-, i-, s- or t-butyl or trifluoromethyl, or phenyl, benzyl, benzoyl, phenoxy, phenylthio, benzyloxy or benzylthio, each of which is optionally monosubstituted to trisubstituted by identical or different substituents from the series consisting of fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy, trifluoromethoxy, methylthio, ethylthio, n- or i-propylthio, n-, i-, s- or t-butylthio, difluoromethylthio or trifluoromethylthio.

3. Azatrioxaspiroalkene of the formula (I) according to Claim 1, in which
Ar¹ represents phenyl which is optionally monosubstituted to disubstituted by identical or different substituents from the series consisting of fluorine, chlorine, bromine, hydroxyl, methyl, ethyl, n- or i-propyl, methoxy, ethoxy, n- or i-propoxy, trifluoromethyl or trifluoromethoxy, and
Ar² represents phenyl which is optionally monosubstituted to trisubstituted by identical or different substituents from the series consisting of fluorine, chlorine, bromine, cyano, nitro, hydroxyl, or methyl, ethyl, n- or i-propyl, n-, i-, s- or t-butyl, n-, i-, s- or t-pentyl, n-, i-, s- or t-hexyl, n- or i-octyl, n- or i-nonyl, n- or i-decyl, n- or i-dodecyl, methoxy, ethoxy, n- or i-propoxy, n-, i-, s- or t-butoxy, n-, i-, s- or t-pentyloxy, n-, i-, s-or t-hexyloxy, n- or i-octyloxy, n- or i-nonyloxy, n- or i-decyloxy, n- or i-dodecyloxy, methylthio, ethylthio, n- or i-propylthio, n-, i-, s- or t-butylthio, n-, i-, s- or t-pentylthio, n-, i-, s- or t-hexylthio, n- or i-octylthio, n- or i-nonylthio, n- or i-decylthio, nor i-dodecylthio, acetyl, propionyl, n- or i-butyroyl, n-, i-, s- or t-valeroyl, methoxycarbonyl, ethoxycarbonyl, n- or i-propoxycarbonyl, n-, i-, s- or t-butoxycarbonyl, n-, i-, s- or t-pentoxycarbonyl, methylenedioxy, ethylenedioxy, each of which is optionally substituted by fluorine and/or chlorine, or cyclohexyl, cyclohexylmethyl or cyclohexyloxy, each of which is optionally substituted by methyl, ethyl, n- or i-propyl, n-, i-, s- or t-butyl, methoxy, ethoxy, n- or i-propoxy, n-, i-, s- or t-butoxy, cyclohexyl or phenyl, or pyridyloxy which is optionally monosubstituted to trisubstituted by identical or different substituents from the series consisting of fluorine, chlorine, bromine, methyl, ethyl, nor i-propyl, n-, i-, s- or t-butyl or trifluoromethyl, or phenyl, benzyl, benzoyl, phenoxy, phenylthio, benzyloxy or benzylthio, each of which is optionally monosubstituted to trisubstituted by identical or different substituents from the series consisting of fluorine, chlorine, bromine, methyl, ethyl, n- or i-propyl, n-, i-, s- or t-butyl, trifluoromethyl, methoxy, ethoxy, n- or i-propoxy, n-, i-, s- or t-butoxy, difluoromethoxy, trifluoromethoxy, methylthio, ethylthio, n- or i-propylthio, n-, i-, s- or t-butylthio, difluoromethylthio or trifluoromethylthio.

4. Process for the preparation of azatrioxaspiroalkenes of the formula (I) according to Claim 1, characterized in that
(a) amino alcohols of the formula (II) 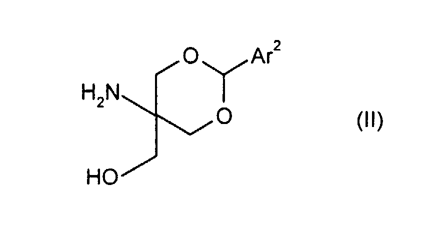 in which
Ar² has the meaning given in claim 1
and carboxylic acids of the general formula (III)
Ar¹-COOH (III)
in which
Ar¹ has the meaning given in Claim 1
are reacted with a dehydrating agent, if appropriate in the presence of a diluent, or in that
(b) amide alcohols of the formula (IV) 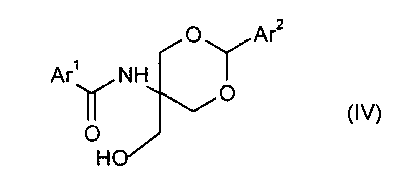 in which
Ar¹ and Ar² have the meanings given in Claim 1
are reacted with a dehydrating agent, if appropriate in the presence of a diluent, or in that
(c) carboxamides of the formula (V) 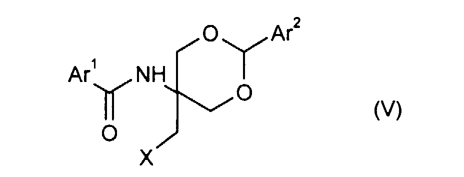 in which
Ar¹ and Ar² have the meanings given in Claim 1 and
X represents halogen, alkylsulphonyloxy or arylsulphonyloxy
are reacted with a base, if appropriate in the presence of a diluent, or in that
(d) carboxamide benzoates of the formula (VI) 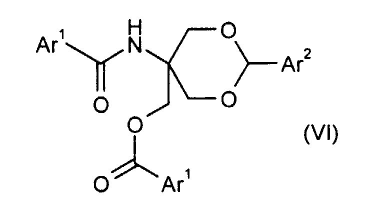 in which
Ar¹ and Ar² have the meanings given in Claim 1
are reacted with a base, if appropriate in the presence of a diluent.

5. Pesticide characterized in that it contains at least one compound of the formula (I) according to Claim 1.

6. Method of combating animal pests, characterized in that compounds of the formula (I) according to Claim 1 are allowed to act on animal pests and/or their environment.

7. Use of compounds of the formula (I) according to Claim 1 for combating animal pests.

8. Process for the preparation of pesticides, characterized in that compounds of the formula (I) according to Claim 1 are mixed with extenders and/or surface-active agents.

9. Amino alcohol of the formula (II) 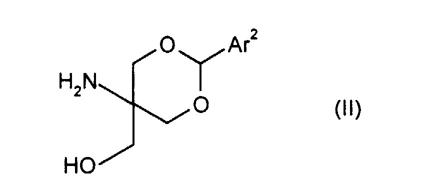 in which
Ar² has the abovementioned meaning, with the exception of the compound of the formula (II) in which Ar² represents phenyl.

10. Amide alcohol of the formula (IV) 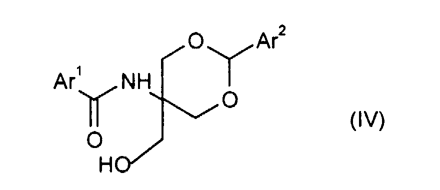 in which
Ar¹ and Ar² are identical or different and have the abovementioned meanings.

11. Carboxamide of the formula (V) 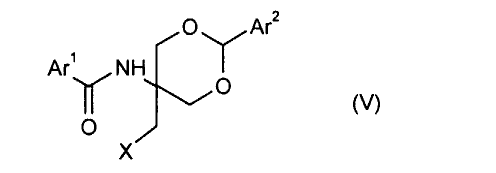 in which
Ar¹ and Ar² are identical or different and have the abovementioned meanings
and
X represents halogen, alkylsulphonyloxy or arylsulphonyloxy.

12. Nitro alcohol of the formula (VII) 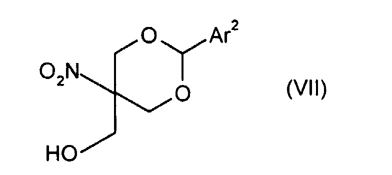 in which
Ar² has the abovementioned meaning with the exception of compounds of the formula (VI) in which Ar² represents phenyl, 4-methoxy-phenyl, 4-cyano-phenyl, 2-nitro-phenyl, 3-nitro-phenyl, 4-nitro-phenyl, 4-chloro-phenyl, 4-dimethylamino-phenyl, 4-hydroxy-phenyl, 2-hydroxyphenyl, 3-ethoxy-4-hydroxy-phenyl or 4-hydroxy-3-methoxy-phenyl.

13. Carboxamide benzoate of the formula (VI) 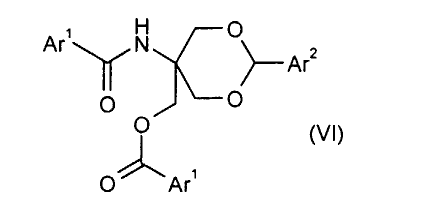 in which
Ar¹ and Ar² have the abovementioned meanings.

## Revendications

1. Azatrioxaspiroalcènes de formule (I) 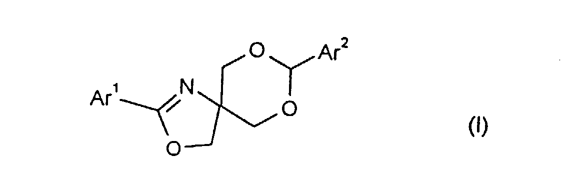 dans laquelle
Ar¹ représente un groupe phényle qui est substitué, le cas échéant, une à cinq fois identiques ou différentes par un halogène, un groupe cyano, nitro, hydroxy, alkyle en C₁ à C₆, alkoxy en C₁ à C₆, alkylthio en C₁ à C₆, halogénalkyle en C₁ à C₆, halogénalkoxy en C₁ à C₆ ou halogénalkylthio en C₁ à C₆,
Ar² représente un groupe phényle qui est substitué, le cas échéant, une à cinq fois identiques ou différentes par un halogène, un groupe cyano, nitro, hydroxy, alkyle en C₁ à C₁₈, halogénalkyle en C₁ à C₈, (alkoxy en C₁ à C₈)-(alkyle en C₁ à C₈), alkoxy en C₁ à C₁₈ (dans lequel, le cas échéant, certains groupes méthylène sont remplacés par des atomes d'oxygène), halogénalkoxy en C₁ à C₈, alkylthio en C₁ à C₁₈, halogénalkylthio en C₁ à C₈, (alkyle en C₁ à C₈)-carbonyle, (halogénalkyle en C₁ à C₈) - carbonyle, (alkoxy en C₁ à C₈)-carbonyle, (halogénalkoxy en C₁ à C₈)-carbonyle, alkylènedioxy en C₁ à C₃, halogénalkylènedioxy en C₁ à C₃, un groupe cyclohexyle, cyclohexylméthyle ou cyclohexyloxy portant chacun, le cas échéant, un substituant alkyle en C₁ à C₆, alkoxy en C₁ à C₆, cyclohexyle ou phényle, un groupe pyridyloxy éventuellement substitué une ou deux fois identiques ou différentes par un halogène, un groupe alkyle en C₁ à C₄ ou halogénalkyle en C₁ à C₄, un groupe phényle, benzyle, benzoyle, phénoxy, phénylthio, benzyloxy ou benzylthio dont chacun est éventuellement substitué une à quatre fois identiques ou différentes par un halogène, un groupe alkyle en C₁ à C₆, halogénalkyle en C₁ à C₆, alkoxy en C₁ à C₆, halogénalkoxy en C₁ à C₆, (alkoxy en C₁ à C₆)-(alkyle en C₁ à C₆), (alkoxy en C₁ à C₆)-éthylèneoxy, alkylthio en C₁ à C₆ ou halogénalkyithio en C₁ à C₆,
leurs mélanges d'isomères ainsi que leurs isomères E ou Z purs.

2. Azatrioxaspiroalcènes de formule générale (I) suivant la revendication 1, dans laquelle
Ar¹ représente un groupe phényle qui est substitué, le cas échéant, une à trois fois identiques ou différentes par du fluor, du chlore, du brome, un groupe cyano, nitro, hydroxy, un groupe méthyle, éthyle, n-propyle, isopropyle, méthoxy, éthoxy, n-propoxy, isopropoxy, méthylthio, éthylthio, n-propylthio ou isopropylthio dont chacun est substitué, le cas échéant par du fluor et/ou du chlore,
Ar² représente un groupe phényle qui est substitué, le cas échéant, une à quatre fois identiques ou différentes par du fluor, du chlore, du brome, un groupe cyano, nitro, hydroxy, un groupe méthyle, éthyle, n-propyle, isopropyle, n-butyle, isobutyle, sec.-butyle, tertio-butyle, n-pentyle, isopentyle, sec.-pentyle, tertiopentyle, n-hexyle, isohexyle, sec.-hexyle, tertio-hexyle, n-octyle, iso-octyle, n-nonyle, isononyle, n-décyle, isodécyle, n-dodécyle, isododécyle, méthoxy, éthoxy, n-propoxy, isopropoxy, n-butoxy, isobutoxy, sec.-butoxy, tertio-butoxy, n-pentyloxy, isopentyloxy, sec.-pentyloxy, tertio-pentyloxy, n-hexyloxy, iso-hexyloxy, sec.-hexyloxy, tertio-hexyloxy, n-octyloxy, iso-octyloxy, n-nonyloxy, isononyloxy, n-décyloxy, isodécyloxy, n-dodécyloxy, iso-dodécyloxy, méthylthio, éthylthio, n-propylthio, isopropylthio, n-butylthio, isobutylthio, sec.-butylthio, tertio-butylthio, n-pentylthio, isopentylthio, sec.-pentylthio, tertio-pentylthio, n-hexylthio, isohexylthio, sec.-hexylthio, tertio-hexylthio, n-octylthio, iso-octylthio, n-nonylthio, isononylthio, n-décylthio, isodécylthio, n-dodécylthio, isododécylthio, acétyle, propionyle, n-butyroyle, isobutyroyle, n-valéroyle, isovaléroyle, sec.-valéroyle, tertiovaléroyle, méthoxycarbonyle, éthoxycarbonyle, n-propoxycarbonyle, isopropoxycarbonyle, n-butoxycarbonyle, isobutoxycarbonyle, sec.-butoxycarbonyle, tertio-butoxycarbonyle, n-pentoxycarbonyle, isopentoxycarbonyle, sec.-pentoxycarbonyle, tertio-pentoxycarbonyle, méthylènedioxy, éthylènedioxy dont chacun est éventuellement substitué par du fluor et/ou du chlore, un groupe cyclohexyle, cyclohexylméthyle ou cyclohexyloxy, dont chacun est éventuellement substitué par un groupe méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, cyclohexyle ou phényle, un groupe pyridyloxy éventuellement substitué une à trois fois identiques ou différentes par du fluor, du chlore, du brome, un groupe méthyle, éthyle, n-propyle, isopropyle, n-butyle, isobutyle, sec.-butyle, tertio-butyle ou par un groupe trifluorométhyle, un groupe phényle, benzyle, benzoyle, phénoxy, phénylthio, benzyloxy ou benzylthio dont chacun est substitué, le cas échéant, une à trois fois identiques ou différentes par du fluor, du chlore, du brome, un groupe méthyle, éthyle, n-propyle, isopropyle, n-butyle, isobutyle, sec.-butyle, tertio-butyle, trifluorométhyle, méthoxy, éthoxy, n-propoxy, isopropoxy, n-butoxy, iso-butoxy, sec.-butoxy, tertio-butoxy, difluorométhoxy, trifluorométhoxy, méthylthio, éthylthio, n-propylthio, isopropylthio, n-butylthio, isobutylthio, sec.-butylthio, tertio-butylthio, difluorométhylthio ou trifluorométhylthio.

3. Azatrioxyspiroalcènes de formule (I) suivant la revendication 1, dans laquelle
Ar¹ représente un groupe phényle qui est substitué, le cas échéant, une ou deux fois identiques ou différentes par du fluor, du chlore, du brome, un groupe hydroxy, méthyle, éthyle, n-propyle, isopropyle, méthoxy, éthoxy, n-propoxy, iso-propoxy, trifluorométhyle ou trifluorométhoxy et
Ar² représente un groupe phényle qui est substitué, le cas échéant, une à trois fois identiques ou différentes par du fluor, du chlore, du brome, un groupe cyano, nitro, hydroxy, un groupe méthyle, éthyle, n-propyle, isopropyle, n-butyle, isobutyle, sec.-butyle, tertio-butyle, n-pentyle, isopentyle, sec.-pentyle, tertiopentyle, n-hexyle, isohexyle, sec.-hexyle, tertio-hexyle, n-octyle, iso-octyle, n-nonyle, isononyle, n-décyle, isodécyle, n-dodécyle, isododécyle, méthoxy, éthoxy, n-propoxy, iso-propoxy, n-butoxy, isobutoxy, sec.-butoxy, tertio-butoxy, n-pentyloxy, isopentyloxy, sec.-pentyloxy, tertio-pentyloxy, n-hexyloxy, iso-hexyloxy, sec.-hexyloxy, tertio-hexyloxy, n-octyloxy, iso-octyloxy, n-nonyloxy, isononyloxy, n-décyloxy, isodécyloxy, n-dodécyloxy, iso-dodécyloxy, méthylthio, éthylthio, n-propylthio, isopropylthio, n-butylthio, isobutylthio, sec.-butylthio, tertio-butylthio, n-pentylthio, isopentylthio, sec.-pentylthio, tertio-pentylthio, n-hexylthio, isohexylthio, sec.-hexylthio, tertio-hexylthio, n-octylthio, iso-octylthio, n-nonylthio, isononylthio, n-décylthio, isodécylthio, n-dodécylthio, isododécylthio, acétyle, propionyle, n-butyroyle, isobutyroyle, n-valéroyle, isovaléroyle, sec.-valéroyle, tertiovaléroyle, méthoxycarbonyle, éthoxycarbonyle, n-propoxycarbonyle, isopropoxycarbonyle, n-butoxycarbonyle, isobutoxycarbonyle, sec.-butoxycarbonyle, tertio-butoxycarbonyle, n-pentoxycarbonyle, isopentoxycarbonyle, sec.-pentoxycarbonyle, tertio-pentoxycarbonyle, méthylènedioxy, éthylènedioxy dont chacun est substitué, le cas échéant, par du fluor et/ou du chlore, un groupe cyclohexyle, cyclohexylméthyle ou cyclohexyloxy, dont chacun est substitué, le cas échéant, par un groupe méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, cyclohexyle ou phényle, un groupe pyridyloxy éventuellement substitué une à trois fois identiques ou différentes par du fluor, du chlore, du brome, un groupe méthyle, éthyle, n-propyle, isopropyle, n-butyle, isobutyle, sec.-butyle, tertio-butyle ou par un groupe trifluorométhyle, un groupe phényle, benzyle, benzoyle, phénoxy, phénylthio, benzyloxy ou benzylthio dont chacun est éventuellement substitué une à trois fois identiques ou différentes par du fluor, du chlore, du brome, un groupe méthyle, éthyle, n-propyle, isopropyle, n-butyle, isobutyle, sec.-butyle, tertio-butyle, trifluorométhyle, méthoxy, éthoxy, n-propoxy, isopropoxy, n-butoxy, iso-butoxy, sec.-butoxy, tertio-butoxy, difluorométhoxy, trifluorométhoxy, méthylthio, éthylthio, n-propylthio, isopropylthio, n-butylthio, isobutylthio, sec.-butylthio, tertio-butylthio, difluorométhylthio ou trifluorométhylthio.

4. Procédé de production d'azatrioxyspiroalcènes de formule (I) suivant la revendication 1, caractérisé en ce que
(a) on fait réagir des amino-alcools de formule (II) 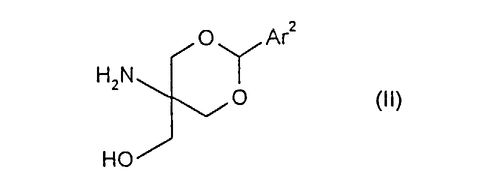 dans laquelle
Ar² a la définition indiquée dans la revendication 1,
avec des acides carboxyliques de formule générale (III)
Ar¹-COOH (III)
dans laquelle
Ar¹ a la définition indiquée dans la revendication 1,
avec un agent déshydratant, le cas échéant, en présence d'un diluant, ou bien
(b) on fait réagir des amido-alcools de formule (IV) 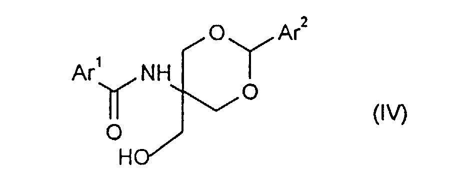 dans laquelle
Ar¹ et Ar² ont la définition indiquée ci-dessus,
avec un agent déshydratant, le cas échéant, en présence d'un diluant, ou bien
(c) on fait réagir des amides d'acides carboxyliques de formule (V) 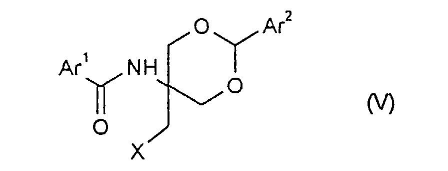 dans laquelle
Ar¹ et Ar² ont la définition indiquée ci-dessus et
X représente un halogène, un groupe alkylsulfonyloxy ou arylsulfonyloxy,
avec une base, le cas échant, en présence d'un diluant, ou bien
(d) on fait réagir des carboxamidobenzoates de formule (VI) 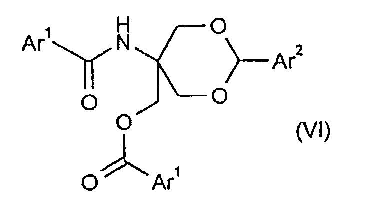 dans laquelle
Ar¹ et Ar² ont la définition indiquée ci-dessus, avec une base, le cas échéant, en présence d'un diluant.

5. Compositions pesticides, caractérisées par une teneur en au moins un composé de formule (I) suivant la revendication 1.

6. Procédé pour combattre des parasites animaux, caractérisé en ce qu'on fait agir des composés de formule (I) suivant la revendication 1 sur des parasites animaux et/ou sur leur milieu.

7. Utilisation de composés de formule (I) suivant la revendication 1 pour combattre des parasites animaux.

8. Procédé de préparation de compositions pesticides, caractérisé en ce qu'on mélange des composés de formule (I) suivant la revendication 1 avec des diluants et/ou des agents tensio-actifs.

9. Amino-alcools de formule (II) 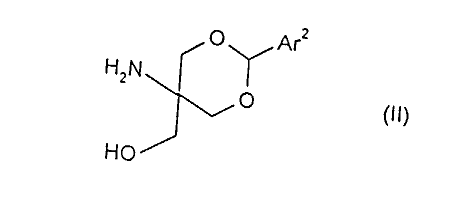 dans laquelle
Ar² a la définition indiquée ci-dessus, à l'exception du composé de formule (II) dans laquelle Ar² est un groupe phényle.

10. Amido-alcools de formule (IV) 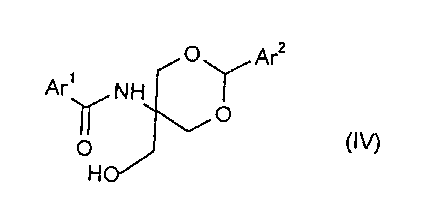 dans laquelle
Ar¹ et Ar² sont identiques ou différents et ont les définitions indiquées ci-dessus.

11. Carboxamides de formule (V) 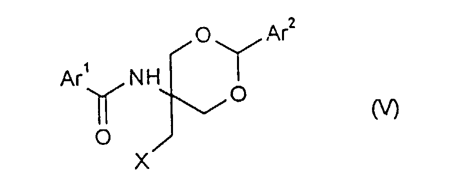 dans laquelle
Ar¹ et Ar² sont identiques ou différents et ont les définitions indiquées ci-dessus
et
X représente un halogène, un groupe alkylsulfonyloxy ou arylsulfonyloxy.

12. Nitro-alcools de formule (VII) 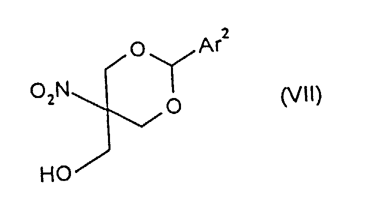 dans laquelle
Ar² a la définition indiquée ci-dessus, à l'exception des composés de formule (VI), dans laquelle Ar² est un groupe phényle, 4-méthoxyphényle, 4-cyanophényle, 2-nitrophényle, 3-nitrophényle, 4-nitrophényle, 4-chlorophényle, 4-diméthylaminophényle, 4-hydroxyphényle, 2-hydroxyphényle, 3-éthoxy-4-hydroxyphényle ou 4-hydroxy-3-méthoxyphényle.

13. Carboxamidobenzoates de formule (VI) 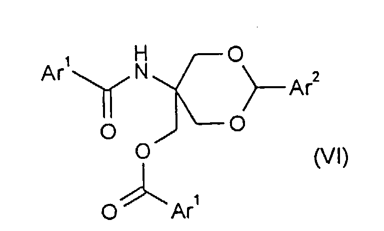 dans laquelle
Ar¹ et Ar² ont la définition indiquée ci-dessus.
